# EUROPÄISCHE PATENTSCHRIFT

(11) **EP 0 758 332 B1**
(45) Veröffentlichungstag und Bekanntmachung des Hinweises auf die Patenterteilung: **07.07.1999**
(21) Anmeldenummer: 95917359.2
(22) Anmeldetag: 24.04.1995
(51) Int. Cl.: C07D 491/048, A01N 43/90, C07F 9/6561, C07D 491/056

(54) **SUBSTITUIERTE BENZIMIDAZOLE ZUR BEKÄMPFUNG VON SCHÄDLINGEN**
SUBSTITUTED BENZIMIDAZOLES FOR PEST CONTROL
BENZIMIDAZOLES SUBSTITUES UTILISES DANS LA LUTTE CONTRE LES PARASITES

(30) Priorität: 06.05.1994 DE 4416116
(43) Veröffentlichungstag der Anmeldung: 19.02.1997
(73) Patentinhaber: BAYER AG, 51368 Leverkusen (DE)
(72) Erfinder: ASSMANN, Lutz, D-23701 Eutin (DE); MARHOLD, Albrecht, D-51373 Leverkusen (DE); DEHNE, Heinz-Wilhelm, D-53125 Bonn (DE); DUTZMANN, Stefan, D-40721 Hilden (DE); ERDELEN, Christoph, D-42799 Leichlingen (DE); STENZEL, Klaus, D-40595 Düsseldorf (DE)
(86) Internationale Anmeldenummer: EP9501536
(87) Internationale Veröffentlichungsnummer: WO9530677

(56) Entgegenhaltungen:
- EP-A- 0 517 476
- EP-A- 0 545 204
- DE-A- 2 014 293
- DE-A- 2 047 369

## Beschreibung

Die vorliegende Erfindung betrifft neue, substituierte Benzimidazole, ein Verfahren zu ihrer Herstellung und ihre Verwendung zur Bekämpfung von Schädlingen.

Es ist bereits bekannt geworden, daß bestimmte Benzimidazol-Derivate fungizide und akarizide Eigenschaften besitzen (vgl. DE-OS 4 139 950 und EP-OS 0 517 476). So lassen sich z.B. 2-Cyano-3 dimethylaminosulfonyl-6,6,7,7-tetrafluor-[1,4]dioxino[2,3-f]benzimidazol sowie 2-Cyano-3-dimethylaminosulfonyl-6,6,7-trifluor-7-chlor-[1,4]dioxino-[2,3-f]benzimidazol oder 2-Cyano-3-dimethylaminosulfonyl-6,7,7-trifluor-6-chlor-[1,4]dioxino[2,3-f]benzimidazol zur Bekämpfung von Pilzen und Akariden einsetzen. Die Wirksamkeit dieser Stoffe ist aber, insbesondere bei niedrigen Aufwandmengen, nicht in allen Fällen völlig befriedigend.

Es wurden nun neue, substituierte Benzimidazole der Formel 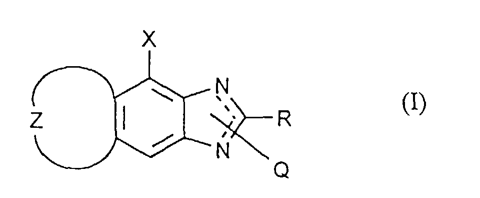 in welcher
- R: für Cyano oder die Gruppierungen 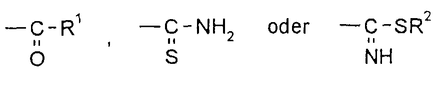 steht, worin
- R¹: für geradkettiges oder verzweigtes Alkyl mit 1 bis 4 Kohlenstoffatomen, geradkettiges oder verzweigtes Alkenyl mit 2 bis 4 Kohlenstoffatomen, geradkettiges oder verzweigtes Alkinyl mit 2 bis 4 Kohlenstoffatomen, geradkettiges oder verzweigtes Halogenalkyl mit 1 bis 4 Kohlenstoffatomen und 1 bis 5 Fluor-, Chlor- und/oder Bromatomen oder für geradkettiges oder verzweigtes Halogenalkenyl mit 2 bis 4 Kohlenstoffatomen und 1 bis 5 Fluor-, Chlor- und/oder Bromatomen steht,
- R²: für geradkettiges oder verzweigtes Alkyl mit 1 bis 4 Kohlenstoffatomen, geradkettiges oder verzweigtes Alkenyl mit 2 bis 4
Kohlenstoffatomen, geradkettiges oder verzweigtes Alkinyl mit 2 bis 4 Kohlenstoffatomen, geradkettiges oder verzweigtes Halogenalkyl mit 1 bis 4 Kohlenstoffatomen und 1 bis 5 Fluor-, Chlor- und/oder
Bromatomen oder für geradkettiges oder verzweigtes Halogenalkenyl mit 2 bis 4 Kohlenstoffatomen und 1 bis 5 Fluor-, Chlor- und/oder Bromatomen steht,
- Q: für die Gruppierung 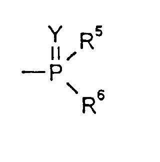 steht, worin
- Y: für Sauerstoff oder Schwefel steht,
- R⁵ und R⁶: unabhängig voneinander für geradkettiges oder verzweigtes Alkyl mit 1 bis 4 Kohlenstoffatomen, geradkettiges oder verzweigtes Alkoxy mit 1 bis 4 Kohlenstoffatomen, geradkettiges oder verzweigtes Alkylthio mit 1 bis 4 Kohlenstoffatomen, geradkettiges oder verzweigtes Alkenyl mit 2 bis 4 Kohlenstoffatomen, geradkettiges oder verzweigtes Alkenyloxy mit 2 bis 4 Kohlenstoffatomen, geradkettiges oder verzweigtes Alkenylthio mit 2 bis 4 Kohlenstoffatomen, geradkettiges oder verzweigtes Alkinyl mit 2 bis 4 Kohlenstoffatomen, geradkettiges oder verzweigtes Alkinyloxy mait 2 bis 4 Kohlenstoffatomen, geradkettiges oder verzweigtes Alkinylthio mit 2 bis 4 Kohlenstoffatomen, Amino, Alkylamino mit 1 bis 4 Kohlenstoffatomen oder für Dialkylamino mit 1 bis 4 Kohlenstoffatomen in jedem Alkylteil stehen,
oder für Phenyl, Phenoxy oder Phenylthio stehen, wobei jeder dieser Reste einfach bis dreifach, gleichartig oder verschieden substituiert sein kann durch Fluor, Chlor, Brom, Alkyl mit 1 bis 4 Kohlenstoffatomen, Halogenalkyl mit 1 bis 4 Kohlenstoffatomen und 1 bis 5 Fluor-, Chlor- und/oder Bromatomen, Alkoxy mit 1 bis 4
Kohlenstoffatomen und/oder Halogenalkoxy mit 1 bis 4 Kohlenstoffatomen und 1 bis 5 Fluor-, Chlor- und/oder Bromatomen,
oder für Cycloalkyl mit 3 bis 7 Kohlenstoffatomen, Cycloalkyloxy mit 3 bis 7 Kohlenstoffatomen, Cycloalkylthio mit 3 bis 7 Kohlenstoffatomen, Cycloalkylamino mit 3 bis 7 Kohlenstoffatomen, Di-cycloalkylamino mit 3 bis 7 Kohlenstoffatomen in jedem Cycloalkylteil, N-Phenyl-N-alkyl-amino mit 1 bis 4 Kohlenstoffatomen im Alkylteil, Pyrrolidinyl, Piperidinyl, Pyrrolinyl, Dihydropyridinyl oder Tetrahydropyridinyl stehen, wobei jeder dieser zuvor genannten Reste einfach bis dreifach, gleichartig oder verschieden substituiert sein kann durch Fluor, Chlor, Brom, Alkyl mit 1 bis 4 Kohlenstoffatomen, Alkoxy mit 1 bis 4 Kohlenstoffatomen und/oder Halogenalkyl mit 1 bis 4 Kohlenstoffatomen und 1 bis 5 Fluor-, Chlor- und/oder Bromatomen, oder
- R⁵ und R⁶: gemeinsam mit dem Phosphoratom, an das sie gebunden sind, für einen 5- oder 6-gliedrigen Heterocyclyl-Rest stehen, der ein oder zwei weitere Heteroatome, wie Sauerstoff, Schwefel und/oder Stickstoff, enthalten kann und einfach bis dreifach, gleichartig oder verschieden substituiert sein kann durch Fluor, Chlor, Brom, Alkyl mit 1 bis 4 Kohlenstoffatomen, Alkoxy mit 1 bis 4 Kohlenstoffatomen und/oder Halogenalkoxy mit 1 bis 4 Kohlenstoffatomen und 1 bis 5 Fluor-, Chlor- und/oder Bromatomen,
- X: für Wasserstoff, Fluor, Chlor oder Brom steht und
- Z: für die Gruppierungen 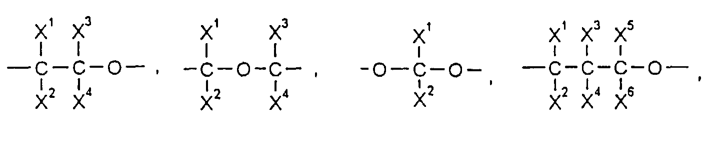 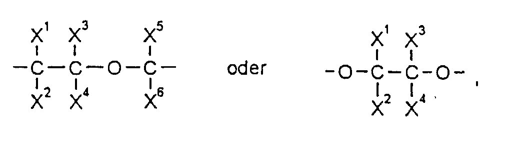 steht, worin
X¹, X³ und X⁵ unabhängig voneinander für Wasserstoff, Fluor, Chlor oder Brom stehen und
X², X⁴ und X⁶ unabhängig voneinander für Fluor, Chlor oder Brom stehen;
gefunden.

Die Verbindungen der Formel (I) können in Abhängigkeit von der Art der Substituenten als geometrische und/oder optische Isomere oder Isomerengemische unterschiedlicher Zusammensetzung vorliegen. Die Erfindung betrifft sowohl die reinen Isomeren als auch die Isomerengemische.

In der Formel (I) steht die gestrichelte Linie für eine Doppelbindung zwischen einem der beiden Stickstoffatome und dem benachbarten Kohlenstoffatom, das den Substituenten R trägt.

Weiterhin wurde gefunden, daß man substituierte Benzimidazole der Formel (I) erhält, wenn man Benzimidazol-Derivate der Formel 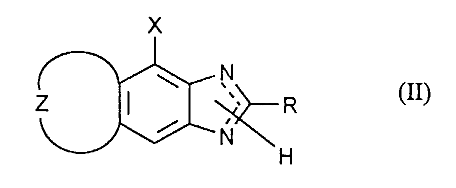 in welcher
- R, X und Z: die oben angegebenen Bedeutungen haben,
mit Chloriden der Formel

Cl-Q (III)

in welcher
- Q: die oben angegebene Bedeutung hat,
gegebenenfalls in Gegenwart eines Verdünnungsmittels und gegebenenfalls in Gegenwart einer anorganischen oder organischen Base umsetzt.

Schließlich wurde gefunden, daß die substituierten Benzimidazole der Formel (I) sehr gut zur Bekämpfung von Schädlingen geeignet sind. Die Stoffe zeichnen sich insbesondere durch hohe fungizide, insektizide und akarizide Wirksamkeit aus.

Überraschenderweise zeigen die erfindungsgemäßen, substituierten Benzimidazole eine bessere Wirksamkeit als 2-Cyano-3-dimethylaminosulfonyl-6,6,7,7-tetrafluor-[1,4]dioxino[2,3-f]benzimidazol, 2-Cyano-3-dimethylaminosulfonyl-6,6,7-trifluor-7-chlor-[1,4]dioxino[2,3-f]benzimidazol und 2-Cyano-3-dimethylaminosulfonyl-6,7,7-trifluor-6-chlor-[1,4]dioxino[2,3-f]benzimidazol, welches konstitutionell ähnliche, vorbekannte Wirkstoffe gleicher Wirkungsrichtung sind.

Die erfindungsgemäßen Stoffe sind durch die Formel (I) allgemein definiert.
- R: steht bevorzugt für Cyano oder für die Gruppierungen 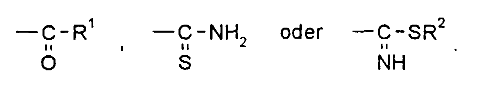
R¹ steht bevorzugt für Methyl, Ethyl, n- oder i-Propyl, n-, i-, s- oder t-Butyl; Allyl, n- oder s-Butenyl; Propargyl, n- oder s-Butinyl; oder für einfach bis dreifach, gleich oder verschieden durch Fluor und/oder Chlor substituiertes Methyl, Ethyl, Allyl und n- oder s-Butenyl.
R² steht bevorzugt für Methyl, Ethyl, n- oder i-Propyl, n-, i-, s- oder t-Butyl; Allyl, n- oder s-Butenyl; Propargyl, n- oder s-Butinyl; oder für einfach bis dreifach, gleich oder verschieden durch Fluor und/oder Chlor substituiertes Methyl, Ethyl, Allyl und n- oder s-Butenyl.
- Q: steht auch bevorzugt für die Gruppierung 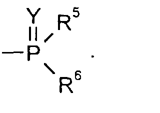
- Y: steht auch bevorzugt für Sauerstoff oder Schwefel.
R⁵ und R⁶ stehen unabhängig voneinander bevorzugt für Methyl, Ethyl, n- oder i-Propyl, n-, i-, s- oder t-Butyl; Methoxy, Ethoxy, n- oder i-Propoxy, n-, i-, s- oder t-Butoxy; Methylthio, Ethylthio, n- oder i-Propylthio; Allyl, n- oder s-Butenyl; Allyloxy, n- oder s-Butenyloxy; Allylthio, n- oder s-Butenylthio; Propargyl, n- oder s-Butinyl; Propargyloxy; Propargylthio; Amino; Methylamino, Ethylamino, n- oder i-Propylamino, n-, i-, s- oder t-Butylamino; Dimethylamino, Diethylamino, Di-n- oder i-Propylamino, Methylethylamino, Methyl-n- oder i-Propylamino; oder
für Phenyl, Phenoxy oder Phenylthio, wobei jeder dieser Reste einfach bis dreifach, gleichartig oder verschieden substituiert sein kann durch Fluor, Chlor, Brom, Methyl, Ethyl, n- oder i-Propyl, Methoxy, Ethoxy, n- oder
i-Propoxy, Trifluormethyl und/oder Trifluormethoxy; oder für Cyclopropyl, Cyclopentyl, Cyclohexyl, Cyclopropyloxy, Cyclopentyloxy, Cyclohexyloxy, Cyclopropylthio, Cyclopentylthio, Cyclohexylthio, Cyclopropylamino, Cyclopentylamino, Cyclohexylamino, Di-cyclohexylamino, N-Phenyl-N-alkylamino mit 1 bis 3 Kohlenstoffatomen im Alkylteil, 1-Pyrrolidinyl, 1-Pyrrolinyl, 1-Piperidinyl, 1-Dihydropyridinyl und 1-Tetrahydropyridinyl, wobei jeder dieser Reste einfach bis dreifach, gleichartig oder verschieden substituiert sein kann durch Fluor, Chlor, Brom, Methyl, Ethyl, n- oder i-Propyl, Methoxy und/oder Trifluormethyl.
- R⁵ und R⁶: stehen außerdem gemeinsam mit dem Phosphoratom, an das sie gebunden sind, bevorzugt für einen 5- oder 6-gliedrigen Heterocyclyl-Rest, der ein oder zwei weitere Heteroatome, wie Sauerstoff, Schwefel und/oder Stickstoff, enthalten kann und einfach bis dreifach, gleichartig oder verschieden substituiert sein kann durch Methyl, Ethyl, n- oder i-Propyl, Methoxy, Ethoxy, n- oder i-Propoxy, Chlor und/oder Trifluormethyl.
- Z: steht bevorzugt für die Gruppierungen -O-CF₂-O-, -O-CF₂-CHF-O-, -O-CHF-CHF-O-, -O-CF₂-CF₂-O-, -O-CF₂-CFCl-O- oder -O-CFCl-CFCl-O-.

Bevorzugte erfindungsgemäße Stoffe sind auch Verbindungen der folgenden Formeln: 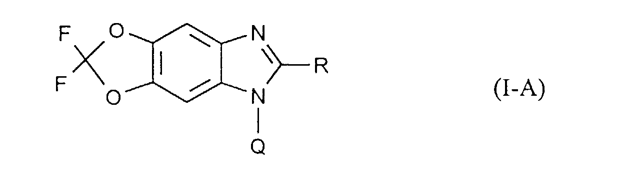 in welcher
- R: für die Gruppierungen -CN, -CSNH₂ oder -COCH₃ steht und
- Q: für die obengenannten Bedeutungen steht. 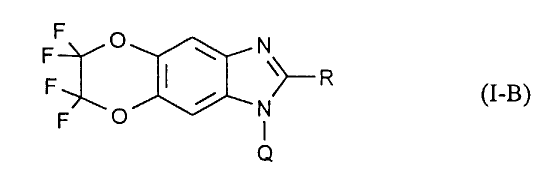
in welcher
- R: für die Gruppierungen -CN, -CSNH₂ oder -COCH₃ steht und
- Q: für die obengenannten Bedeutungen steht. 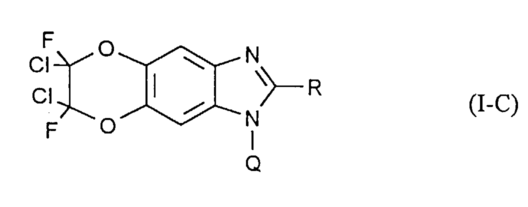
in welcher
- R: für die Gruppierungen -CN, -CSNH₂ oder -COCH₃ steht und
- Q: für die obengenannten Bedeutungen steht.

Beispielhaft seien für den Substituenten Q folgende Reste genannt:

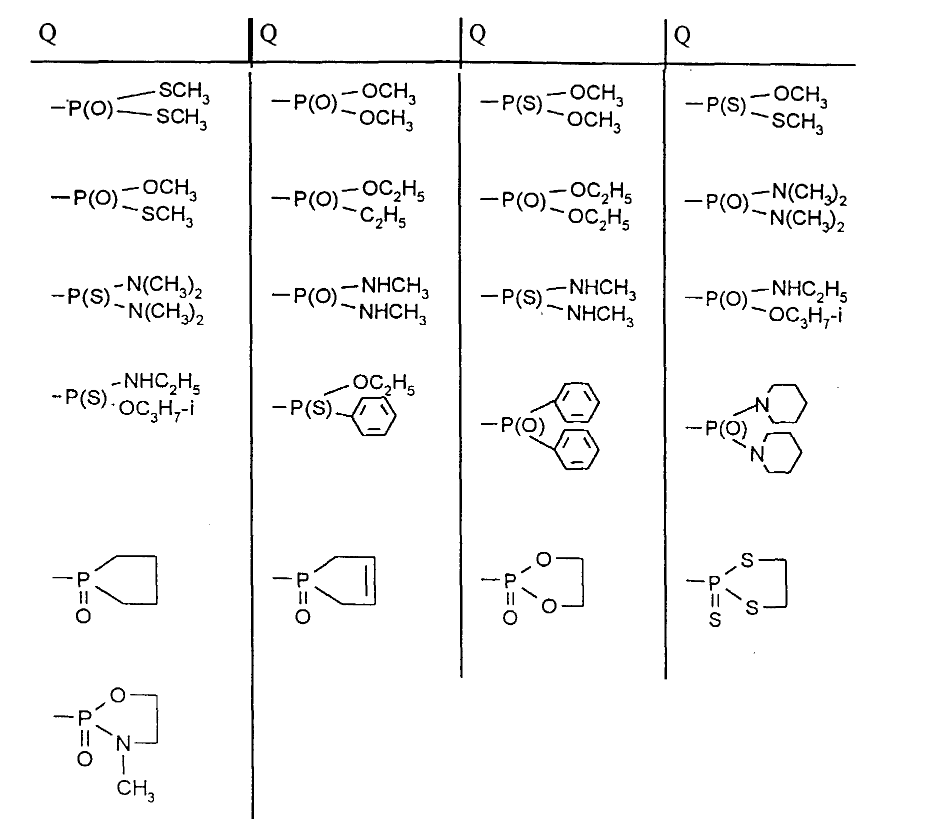

Die zur Durchführung des erfindungsgemäßen Verfahrens als Ausgangsstoffe benötigten Benzimidazol-Derivate sind durch die Formel (II) allgemein definiert. In der Formel (II) haben R, X und Z bevorzugt diejenigen Bedeutungen, die bereits oben im Zusammenhang mit der Beschreibung der Verbindungen der Formel (I) als bevorzugt für R, X und Z angegeben wurden.

Die Benzimidazol-Derivate der Formel (II) sind bekannt bzw. in allgemein bekannter Art und Weise erhältlich (vgl. z.B. EP-A 0 517 476, DE-OS 4 139 950, FR-A 2 607 811, EP-A 0 549 943 sowie die Herstellungsbeispiele).

Die außerdem zur Durchführung des erfindungsgemäßen Verfahrens als Ausgangsstoffe benötigten Chloride sind durch die Formel (III) allgemein definiert. In der Formel (III) hat Q bevorzugt diejenigen Bedeutungen, die bereits oben im Zusammenhang mit der Beschreibung der Verbindungen der Formel (I) als bevorzugt für Q angegeben wurden.

Als Verdünnungsmittel kommen bei der Durchführung des erfindungsgemäßen Verfahrens alle üblichen inerten, organischen Solventien in Betracht. Vorzugsweise verwendbar sind aliphatische, cycloaliphatiche und aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform oder Tetrachlorkohlenstoff; außerdem Ether, wie Diethylether, Diisopropylether, Dioxan, Tetrahydrofuran oder Ethylenglykoldimethyl- oder -diethylether, ferner Ketone, wie Aceton oder Butanon oder Methyl-isobutyl-keton; Nitrile wie Acetronitril, Propionitril oder Benzonitril, oder Ester wie Essigsäuremethylester oder Essigsäureethylester.

Das erfindungsgemäße Verfahren wird vorzugsweise in Gegenwart einer anorganischen oder organischen Base durchgeführt.

Vorzugsweise verwendbar sind Erdalkali- oder Alkalimetallhydroxide wie Natriumhydroxid, Calciumhydroxid, Kaliumhydroxid, oder auch Ammoniumhydroxid, Alkalimetallcarbonate, wie Natriumcarbonat, Kaliumcarbonat, Kaliumhydrogencarbonat, Natriumhydrogencarbonat, Alkali- oder Erdalkalimetallacetate wie Natriumacetat, Kaliumacetat, Calciumacetat, sowie tertiäre Amine, wie Trimethylamin, Triethylamin, Tributylamin, N,N-Dimethylanilin, Pyridin, N-methylpiperidin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens in einem größeren Bereich variiert werden. Im allgemeinen arbeitet man bei Temperaturen zwischen 0 und 150°C, vorzugsweise bei Temperaturen zwischen 20 und 120°C.

Das erfindungsgemäße Verfahren wird üblicherweise unter Normaldruck durchgeführt. Es ist jedoch auch möglich, unter erhöhtem oder vermindertem Druck zu arbeiten.

Zur Durchführung des erfindungsgemäßen Verfahrens setzt man pro Mol an Benzimidazol-Derivat der Formel (II) in einem Verdünnungsmittel im allgemeinen 1,0 bis 2,0 Mol, vorzugsweise 1,0 bis 1,3 Mol an Chlorid der Formel (III) und gegebenenfalls 1,0 bis 2,0 Mol, vorzugsweise 1,0 bis 1,3 Mol an Base ein. Die Reaktionsführung, Aufarbeitung und Isolierung der Reaktionsprodukte erfolgt nach bekannten Verfahren (vgl. auch die Herstellungsbeispiele).

Die erfindungsgemäßen Wirkstoffe der Formel (I) weisen eine starke Wirkung gegen Schädlinge auf und können zur Bekämpfung von unerwünschten Schadorganismen praktisch eingesetzt werden. Die Wirkstoffe sind für den Gebrauch als Pflanzenschutzmittel, insbesondere als Fungizide, Insektizide und Akarizide geeignet.

Fungizide werden im Pflanzenschutz eingesetzt zur Bekämpfung von Plasmodiophoromycetes, Oomycetes, Chytridiomycetes, Zyomycetes, Ascomycetes, Basidiomycetes und Deuteromycetes.

Beispielhaft aber nicht begrenzend seien einige Erreger von pilzlichen Erkrankungen, die unter die oben aufgezählten Oberbegriffe fallen, genannt:
Pythium-Arten, wie beispielsweise Pythium ultimum;
Phytophthora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus (Konidienform: Drechlera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellucularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei können die erfindungsgemäßen Wirkstoffe mit besonders gutem Erfolg zur Bekämpfung von Krankheiten im Obst- und Gemüsebau, wie beispielsweise gegen Venturia-Arten oder zur Bekämpfung von Getreidekrankheiten, wie beispielsweise gegen Erysiphe-, Cochliobolus-, Pyrenophora- oder Septoria-Arten oder zur Bekämpfung von Reis-Krankheiten, wie beispielsweise gegen den Erreger der Reisfleckenkrankheit (Pyricularia oryzae) eingesetzt werden.

Darüber hinaus eignen sich die erfindungsgemäßen Wirkstoffe zur Bekämpfung von tierischen Schädlingen, vorzugsweise Arthropoden und Nematoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:
Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Bruchidius obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.
Aus der Ordnung der Siphonaptera z.B. Xenopsylla cheopis, Ceratophyllus spp..
Aus der Ordnung der Arachnida z.B. Scorpio maurus, Latrodectus mactans.
Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..
Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp..

Die erfindungsgemäßen Wirkstoffe der Formel (I) zeichnen sich auch durch hervorragende insektizide Wirkung aus, z.B. gegen Phaedon-, Spodoptera-, Nephotettix- und Mycus-Arten. Sie zeigen aber auch eine gute akarizide Wirkung.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt-und-Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächen-aktiven Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokusnußschalen, Maiskolben und Tabakstengel; als Emulgier- und/oder schaumerzeugende Mittel kommen in Frage: z.B. nichtionogene ond anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexföromige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetable Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können bei der Anwendung als Fungizide als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Besonders günstige Mischpartner sind z.B. die folgenden Verbindungen:

### Fungizide:

2-Aminobutan;2-Anilino-4-methyl-6-cyclopropyl-pyrimidin;2',6'-Dibromo-2-methyl-4'-trifluoromethoxy-4'-trifluoro-methyl-1,3-thiazole-5-carboxanilid;2,6-Dichloro-N-(4-trifluoromethylbenzyl)-benzamid; (E)-2-Methoximino-N-methyl-2-(2-phenoxyphenyl) -acetamid; 8-Hydroxychinolinsulfat;Methyl-(E)-2-{2-[6-(2-cyanophenoxy)-pyrimidin-4-yloxy]-phenyl}-3-methoxyacrylat;Methyl-(E)-methoximino [alpha-(o-tolyloxy)-o-tolyl]-acetat; 2-Phenylphenol (OPP), Aldimorph, Ampropylfos, Anilazin, Azaconazol,
Benalaxyl, Benodanil, Benomyl, Binapacryl, Biphenyl, Bitertanol, Blasticidin-S, Bromuconazole, Bupirimate, Buthiobate,
Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
Dichlorophen, Diclobutrazol, Diclofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodine, Drazoxolon,
Edifenphos, Epoxyconazole, Ethirimol, Etridiazol,
Fenarimol, Fenbuconazole, Fenfuram, Fenitropan, Fenpiclonil, Fenpropidin, Fenpropimorph, Fentinacetate, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
Guazatine,
Hexachlorobenzol, Hexaconazol, Hymexazol,
Imazalil, Imibenconazol, Iminoctadin, Iprobenfos (IBP), Iprodion, Isoprothiolan,
Kasugamycin, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
Mancopper, Mancozeb, Maneb, Mepanipyrim, Mepronil, Metalaxyl, Metconazol, Methasulfocarb, Methfuroxam, Metiram, Metsulfovax, Myclobutanil,
Nickel-dimethyldithiocarbamat, Nitrothal-isopropyl, Nuarimol,
Ofurace, Oxadixyl, Oxamocarb, Oxycarboxin,
Pefurazoat, Penconazol, Pencycuron, Phosdiphen, Pimaricin, Piperalin, Polyoxin, Probenazol, Prochloraz, Procymidon, Propamocarb, Propiconazole, Propineb, Pyrazophos, Pyrifenox, Pyrimethanil, Pyroquilon,
Quintozen (PCNB),
Schwefel und Schwefel-Zubereitungen,
Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
Validamycin A, Vinclozolin,
Zineb, Ziram

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, AC 303 630, Acephat, Acrinathrin, Alanycarb, Aldicarb, Alphamethrin, Amitraz, Avermectin, AZ 60541, Azadirachtin, Azinphos A, Azinphos M, Azocyclotin,
Bacillus thuringiensis, Bendiocarb, Benfuracarb, Bensultap, Betacyfluthrin, Bifenthrin, BPMC, Brofenprox, Bromophos A, Bufencarb, Buprofezin, Butocarboxin, Butylpyridaben,
Cadusafos, Carbaryl, Carbofuran, Carbophenothion, Carbosulfan, Cartap, CGA 157 419, CGA 184699, Chloethocarb, Chlorethoxyfos, Chlorfenvinphos, Chlorfluazuron, Chlormephos, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat,
Dimethylvinphos, Dioxathion, Disulfoton,
Edifenphos, Emamectin, Esfenvalerat, Ethiofencarb, Ethion, Ethofenprox, Ethoprophos, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonophos, Formothion, Fosthiazat, Fubfenprox, Furathiocarb,
HCH, Heptenophos, Hexaflumuron, Hexythiazox,
Imidacloprid, Iprobenfos, Isazophos, Isofenphos, Isoprocarb, Isoxathion, Ivemectin,
Lambda-cyhalothrin, Lufenuron,
Malathion, Mecarbam, Mervinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184, NI 25, Nitenpyram
Omethoat, Oxamyl, Oxydemethon M, Oxydeprofos,
Parathion A, Parathion M, Permethrin, Phenthoat, Phorat, Phosalon, Phosmet, Phosphamdon, Phoxim, Pirimicarb, Pirimiphos M, Pirimiphos A, Profenophos, Promecarb, Propaphos, Propoxur, Prothiophos, Prothoat, Pymetrozin, Pyrachlophos, Pyradaphenthion, Pyresmethrin, Pyrethrum, Pyridaben, Pyrimidifen, Pyriproxifen,
Quinalphos,
RH 5992,
Salithion, Sebufos, Silafluofen, Sulfotep, Sulprofos,
Tebufenozid, Tebufenpyrad, Tebupirimiphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, Zetamethrin.

Auch eine Mischung mit anderen bekannten Wirkstoffen, wie Herbiziden oder mit Düngemitteln und Wachstumsregulatoren ist möglich.

Die Wirkstoffe können bei der Anwendung als Fungizide als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.

Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut von Pflanzen behandelt werden.

Bei der Behandlung von Pflanzenteilen können die Wirkstoffkonzentrationen in den Aufwendungsformen bei der Anwendung als Fungizide in einem größeren Bereich variiert werden. Sie liegen im allgemeinen zwischen 1 und 0,0001 Gew.-%, vorzugsweise zwischen 0,5 und 0,001%.

Bei der Saatgutbehandlung werden bei der Anwendung als Fungizide im allgemeinen Wirkstoffmengen von 0,001 bis 50 g je Kilogramm Saatgut, vorzugsweise 0,01 bis 10g benötigt.

Bei Behandlung des Bodens sind bei der Anwendung als Fungizide Wirkstoffkonzentrationen von 0,00001 bis 0,1 Gew.-%, vorzugsweise von 0,0001 bis 0,02 % am Wirkungsort erforderlich.

Die erfindungsgemäßen Wirkstoffe können bei der Anwendung als Insektizide und Akarizide in ihren handelsüblichen Formulierungen sowie in den aus den Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a..

Genannt seien die folgenden Verbindungen:
Acrinathrin, Alphamethrin, Betacyfluthrin, Bifenthrin, Brofenprox, Cis-Resmethrin, Clocythrin, Cycloprothrin, Cyfluthrin, Cyhalothrin, Cypermethrin, Deltamethrin, Esfenvalerate, Etofenprox, Fenpropathrin, Fenvalerate, Flucythrinate, Fluvalinate, Lambda-Cyhalothrin, Permethrin, Pyresmethrin, Pyrethrum, Silafluofen, Tralomethrin, Zetamethrin,
Alanycarb, Bendiocarb, Benfuracarb, Bufencarb, Butocarboxim, Carbaryl, Cartap, Ethiofencarb, Fenobucarb, Fenoxycarb, Isoprocarb, Methiocarb, Methomyl, Metolcarb, Oxamyl, Pirimicarb, Promecarb, Propoxur, Terbam, Thiodicarb, Thiofanox, Trimethacarb, XMC, Xylylcarb,
Acephate, Azinphos A, Azinphos M, Bromophos A, Cadusafos, Carbophenothion, Chlorfenvinphos, Chlormephos, Chlorpyrifos, Chlorpyrifos M, Cyanophos. Demeton M, Demeton-S-methyl, Demeton S, Diazinon, Dichlorvos, Dicliphos, Dichlorfenthion, Dicrotophos, Dimethoate, Dimethylvinphos, Dioxathion, Disulfoton, Edifenphos, Ethion, Etrimphos, Fenitrothion, Fenthion, Fonophos, Formothion, Heptenophos, Iprobenfos, Isazophos, Isoxathion, Phorate, Malathion, Mecarbam, Mervinphos, Mesulfenphos, Methacrifos, Methamidophos, Naled, Omethoate, Oxydemeton M, Oxydeprofos, Parathion A, Parathion M, Phenthoate, Phorate, Phosalone, Phosmet, Phosphamdon, Phoxim, Pirimiphos A, Pirimiphos M, Propaphos, Prothiophos, Prothoate, Pyridaphenthion, Quinalphos, Salithion, Sebufos, Sulfotep, Sulprofos, Tetrachlorvinphos, Temephos, Thiomethon, Thionazin, Trichlorfon, Triazophos, Vamidothion,
Buprofezin, Chlorfluazuron, Diflubenzuron, Flucycloxuron, Flufenoxuron, Hexaflumuron, Pyriproxifen, Tebufenozide, Teflubenzuron, Triflumuron,
Imidacloprid, Nitenpyram, N-[(6-Chloro-3-pyridinyl)methyl]-N'-cyano-N-methylethanimidamid (NI-25),
Abamectin, Amitrazin, Avermectin, Azadirachtin, Bensultap, Bacillus thuringiensis, Cyromazine, Diafenthiuron, Emamectin, Ethofenprox, Fenpyrad, Fipronil, Flufenprox, Lufenuron, Metaldehyd, Milbemectin, Pymetrozine, Tebufenpyrad, Triazuron,
Aldicarb, Bendiocarb, Benfuracarb, Carbofuran, Carbosulfan, Chlorethoxyfos, Cloethocarb, Disulfoton, Ethophrophos, Etrimphos, Fenamiphos, Fipronil, Fonofos, Fosthiazate, Furathiocarb, HCH, Isazophos, Isofenphos, Methiocarb, Monocrotophos, Nitenpyram, Oxamyl, Phorate, Phoxim, Prothiofos, Pyrachlofos, Sebufos, Silafluofen, Tebupirimphos, Tefluthrin, Terbufos, Thiodicarb, Thiafenox,
Azocyclotin, Butylpyridaben, Clofentezine, Cyhexatin, Diafenthiuron, Diethion, Emamectin, Fenazaquin, Fenbutatin Oxide, Fenothiocarb, Fenpropathrin, Fenpyrad, Fenpyroximate, Fluazinam, Fluazuron, Flucycloxuron, Flufenoxuron, Fluvalinate, Fubfenprox, Hexythiazox, Ivemectin, Methidathion, Monocrotophos, Moxidectin, Naled, Phosalone, Profenofos, Pyridaben, Pyrimidifen, Tebufenpyrad, Thuringiensin, Triarathenesowie4-Bromo-2-(4-chlorophenyl)-1-(ethoxymethyl)-5-(trifluoromethyl)-1H-pyrrole-3-carbonitril (AC 303630).

Die erfindungsgemäßen Wirkstoffe können bei der Anwendung als Insektizide und Akarizide ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv sein muß.

Der Wirkstoffgehalt der aus den handelsüblichen Formulierungen bereiteten Anwendungsformen kann bei der Anwendung als Insektizide und Akarizide in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Die Herstellung und die Verwendung der erfindungsgemäßen Wirkstoffe gehen aus den nachfolgenden Beispielen hervor.

### Herstellungsbeispiele

### Beispiel 1

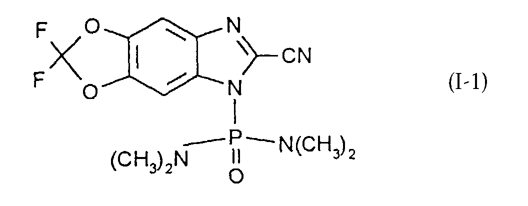

Ein Gemisch aus 4,4 g (20 mMol) 2-Cyano-6,6-difluor-[1,3]dioxolo[4,5-f]benzimidazol und 20 ml Triethylamin wird mit 5,1 g (30 mMol) Phosphorsäure-bis(dimethylamid)-chlorid versetzt und dann 16 Stunden bei 60°C gerührt. Danach wird das Reaktionsgemisch auf Wasser gegossen und dreimal mit je 70 ml Diethylether extrahiert. Die vereinigten organischen Extrakte werden über Natriumsulfat getrocknet und unter vermindertem Druck eingeengt. Der verbleibende Rückstand wird mit einer Mischung aus 5 ml Diethylether und 5 ml Petrolether verrührt. Der dabei anfallende Feststoff wird abfiltriert und getrocknet.

Man erhält 5,8 g (81 % der Theorie) 2-Cyano-6,6-difluor-3-[bis(dimethylamino)phosphinyl]-[1,3]dioxolo[4,5-f]benzimidazol vom Schmelzpunkt 90-93°C.

### Herstellung des Ausgangsproduktes

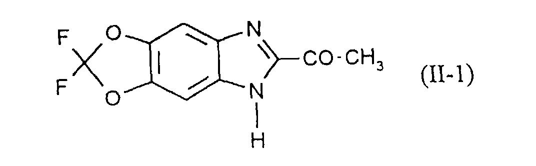

Ein Gemisch aus 18,8 g (0,1 Mol) 5,6-Diamino-2,2-difluor-1,3-benzodioxolan und 150 ml Ethanol wird bei Raumtemperatur unter Rühren mit 11,6 g (0,1 Mol) Brenztraubensäure-ethylester versetzt und dann 48 Stunden unter Rückfluß erhitzt. Aus der erkalteten Reaktionsmischung wird anschließend der Niederschlag abfiltriert, mit 30 ml Petrolether gewaschen und getrocknet.

Man erhält 16,9 g (70 % der Theorie) 2-Acetyl-6,6-difluor-[1,3]dioxolo[4,5-f]benzimidazol vom Schmelzpunkt >220°C.

In entsprechender Weise und gemäß den allgemeinen Angaben zur Herstellung erhält man die folgenden Benzimidazole der Formel

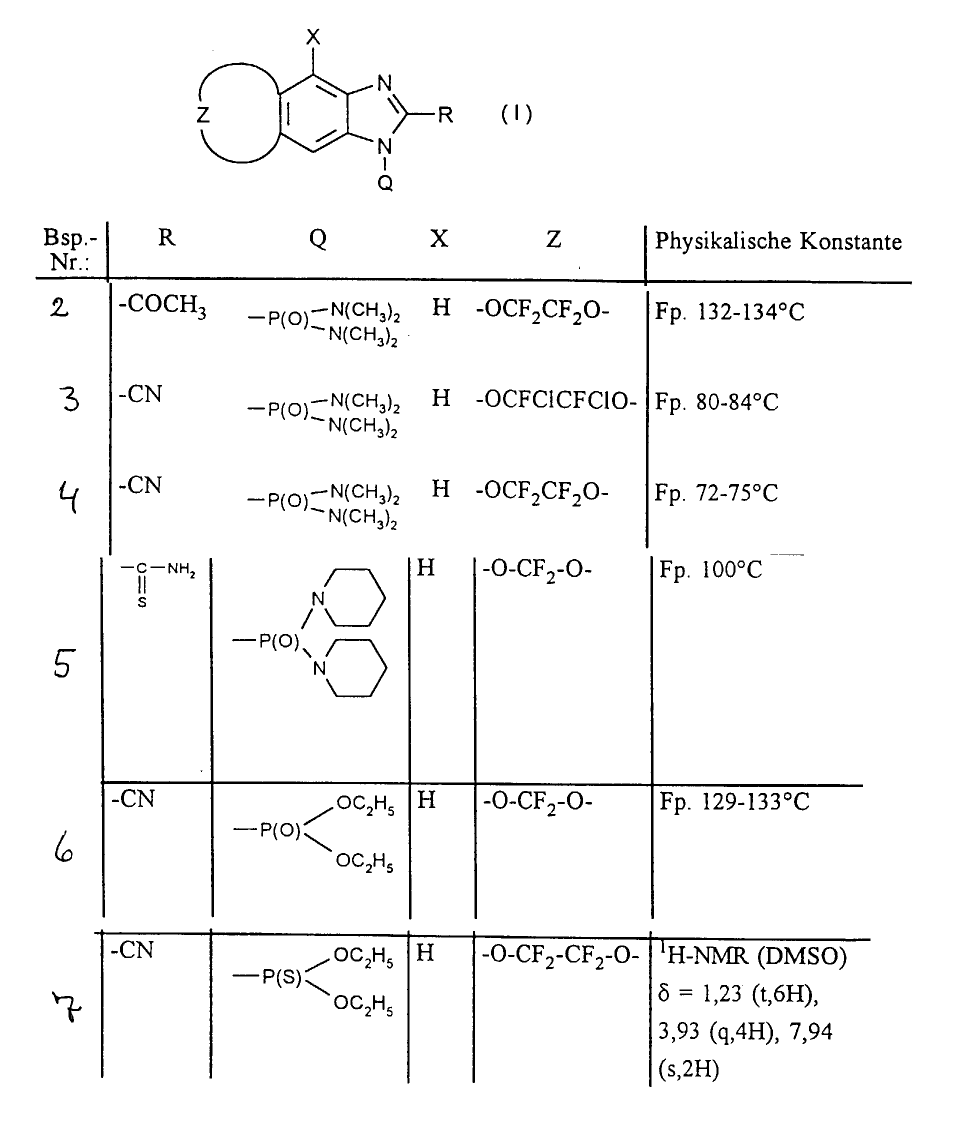

### Anwendungsbeispiele

In den folgenden Anwendungsbeispielen wurden die nachstehend aufgeführten Verbindungen als Vergleichssubstanzen eingesetzt. 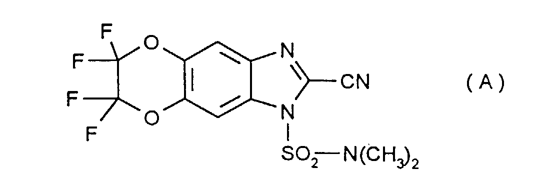 2-Cyano-3-dimethylaminosulfonyl-6,6,7,7-tetrafluor-[1,4]dioxino[2,3-f]benzimidazol 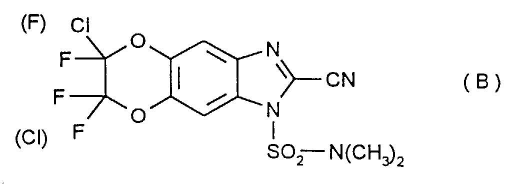 2-Cyano-3-dimethylaminosulfonyl-6,6,7 (bzw. 6,7,7)-trifluor-7 (bzw. 6)-chlor-[1,4] dioxino[2,3 -f]benzimidazol

(Beide bekannt aus EP-A 0 517 476 bzw. DE-OS 4 139 950.)

### Beispiel A

### Erysiphe-Test (Gerste) / protektiv

| | |
|---|---|
| Lösungsmittel | 10 Gewichtsteile N-Methyl-pyrrolidon |
| Emulgator | 0,6 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung in der angegebenen Aufwandmenge.

Nach Antrocknen des Spritzbelages werden die Pflanzen mit Sporen von Erysiphe graminis f.sp. hordei bestäubt.

Die Pflanzen werden in einem Gewächshaus bei einer Temperatur von ca. 20°C und bei einer relativen Luftfeuchtigkeit von ca. 80% aufgestellt, um die Entwicklung von Mehltaupilzen zu begünstigen.

7 Tage nach der Inokulation erfolgt die Auswertung.

Bei einer Aufwandmenge von 125 g/ha zeigt die Verbindung gemäß Beispiel 4 einen Wirkungsgrad von 100 %, während die Vergleichssubstanzen (A) und (B) einen Wirkungsgrad von 83 % aufweisen.

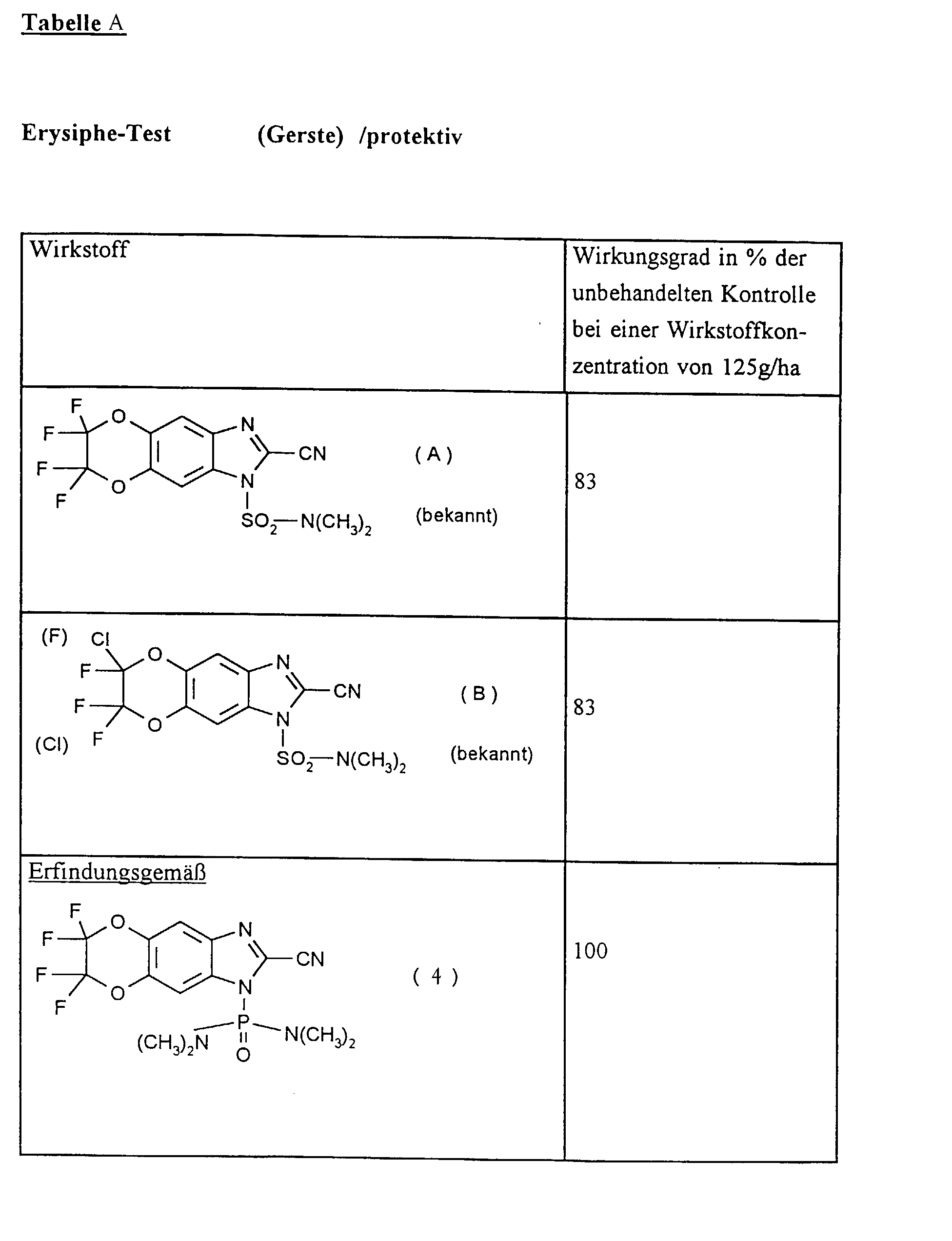

### Beispiel B

### Erysiphe-Test (Weizen)/protektiv

| | |
|---|---|
| Lösungsmittel | 10 Gewichtsteile N-Methyl-pyrrolidon |
| Emulgator | 0,6 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung in der angegebenen Aufwandmenge.

Nach Antrocknen des Spritzbelages werden die Pflanzen mit Sporen von Erysiphe graminis f.sp. tritici bestäubt.

Die Pflanzen werden in einem Gewächshaus bei einer Temperatur von ca. 20°C und bei einer relativen Luftfeuchtigkeit von ca. 80% aufgestellt, um die Entwicklung von Mehltaupusteln zu begünstigen.

7 Tage nach der Inokulation erfolgt die Auswertung.

Bei einer Aufwandmenge von 125 g/ha zeigt die Verbindung gemäß Beispiel 4 einen Wirkungsgrad von 75 %, während die Vergleichssubstanzen (A) und (B) einen Wirkungsgrad von 25 % aufweisen.

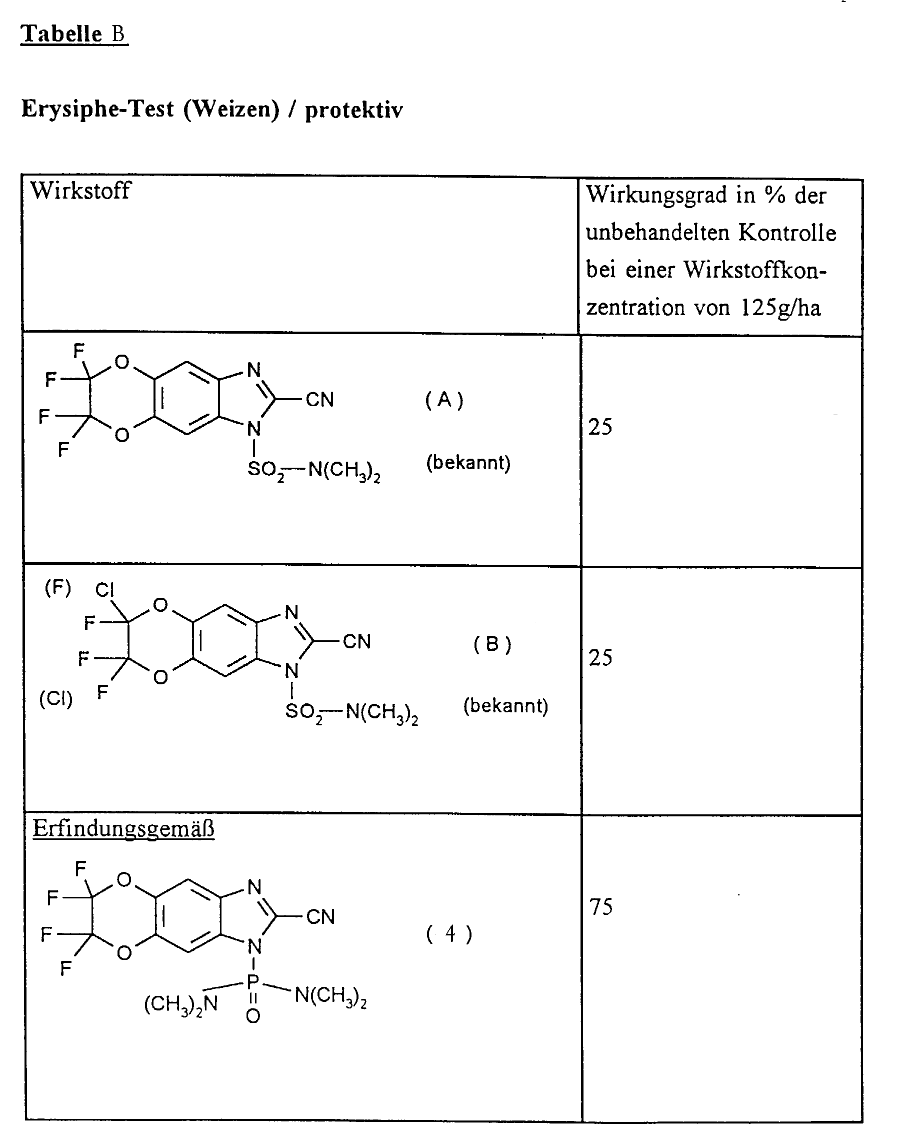

### Beispiel C

### Phaedon-Larven-Test

| | |
|---|---|
| Lösungsmittel | 7 Gewichtsteile Dimethylformamid |
| Emulgator | 1 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Kohlblätter (Brassica oleracea) werden durch Tauchen in die Wirkstoffzubereitung der gewünschten Konzentration behandelt und mit Meerrettichblattkäfer-Larven Phaedon cochleariae besetzt, solange die Blätter noch feucht sind.

Nach der gewünschten Zeit wird der Abtötungsgrad in % bestimmt. Dabei bedeutet 100%, daß alle Käfer-Larven abgetötet wurden; 0% bedeutet, daß keine Käfer-Larven abgetötet wurden.

In diesem Test zeigt die Verbindung gemäß Herstellungsbeispiel 1 bei einer Wirkstoffkonzentration von 0,001% nach 7 Tagen einen Abtötungsgrad von 100%, während die Vergleichssubstanzen (A) und (B) keine Wirkung aufweisen.

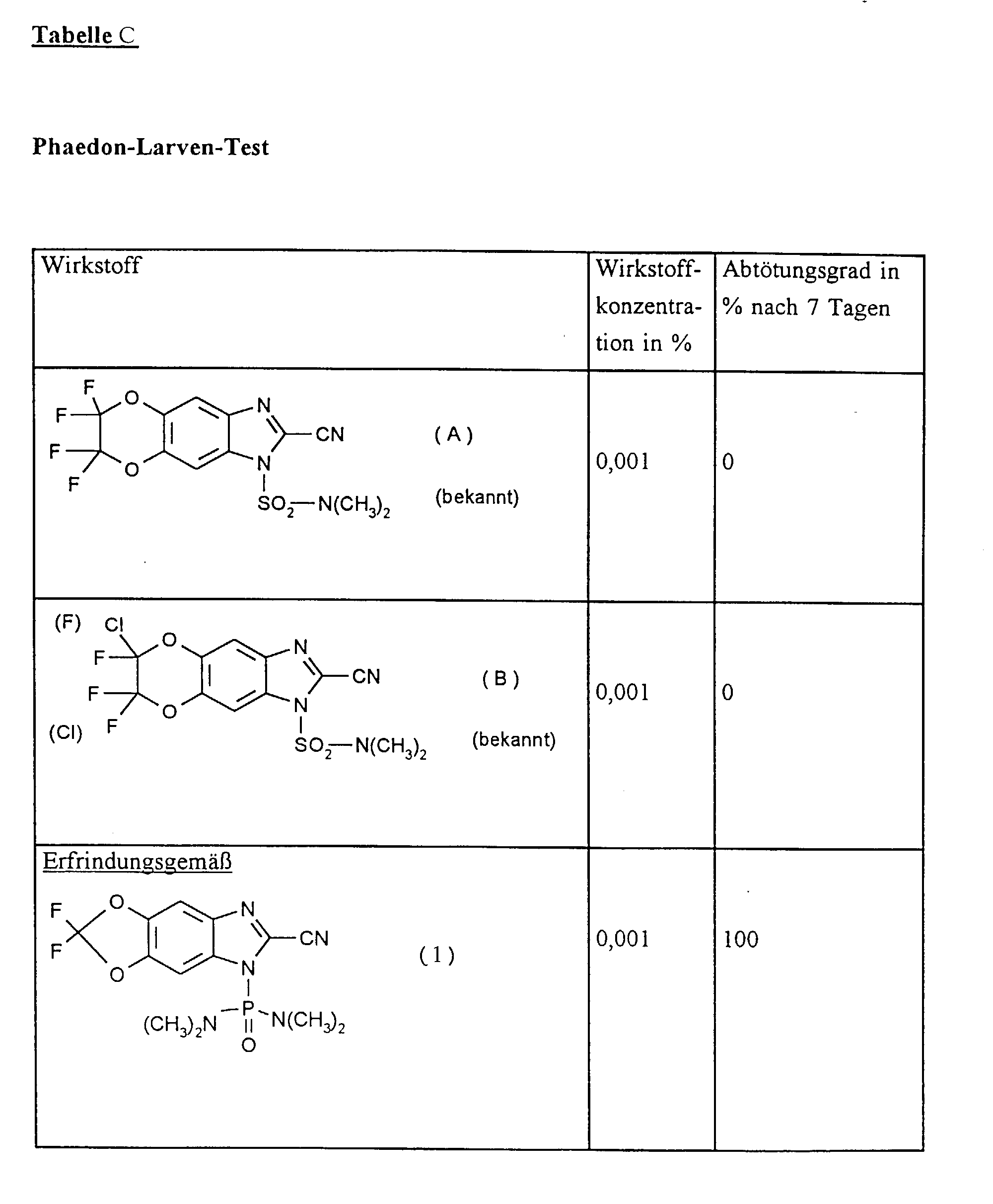

### Beispiel D

### Spodoptera-Test

| | |
|---|---|
| Lösungsmittel | 7 Gewichtsteile Dimethylformamid |
| Emulgator | 1 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Kohlblätter (Brassica oleracea) werden durch Tauchen in die Wirkstoffzubereitung der gewünschten Konzentration behandelt und mit Raupen des Eulenfalters Spodoptera frugiperda besetzt, solange die Blätter noch feucht sind.

Nach der gewünschten Zeit wird der Abtötungsgrad in % bestimmt. Dabei bedeutet 100%, daß alle Raupen abgetötet wurden; 0% bedeutet, daß keine Raupen abgetötet wurden.

In diesem Test zeigt die Verbindung gemäß Herstellungsbeispiel 3 bei einer Wirkstoffkonzentration von 0,01% nach 3 Tagen einen Abtötungsgrad von 100%, während die Vergleichssubstanzen (A) und (B) einen Wirkungsgrad von 10 % bzw. 0 % aufweisen.

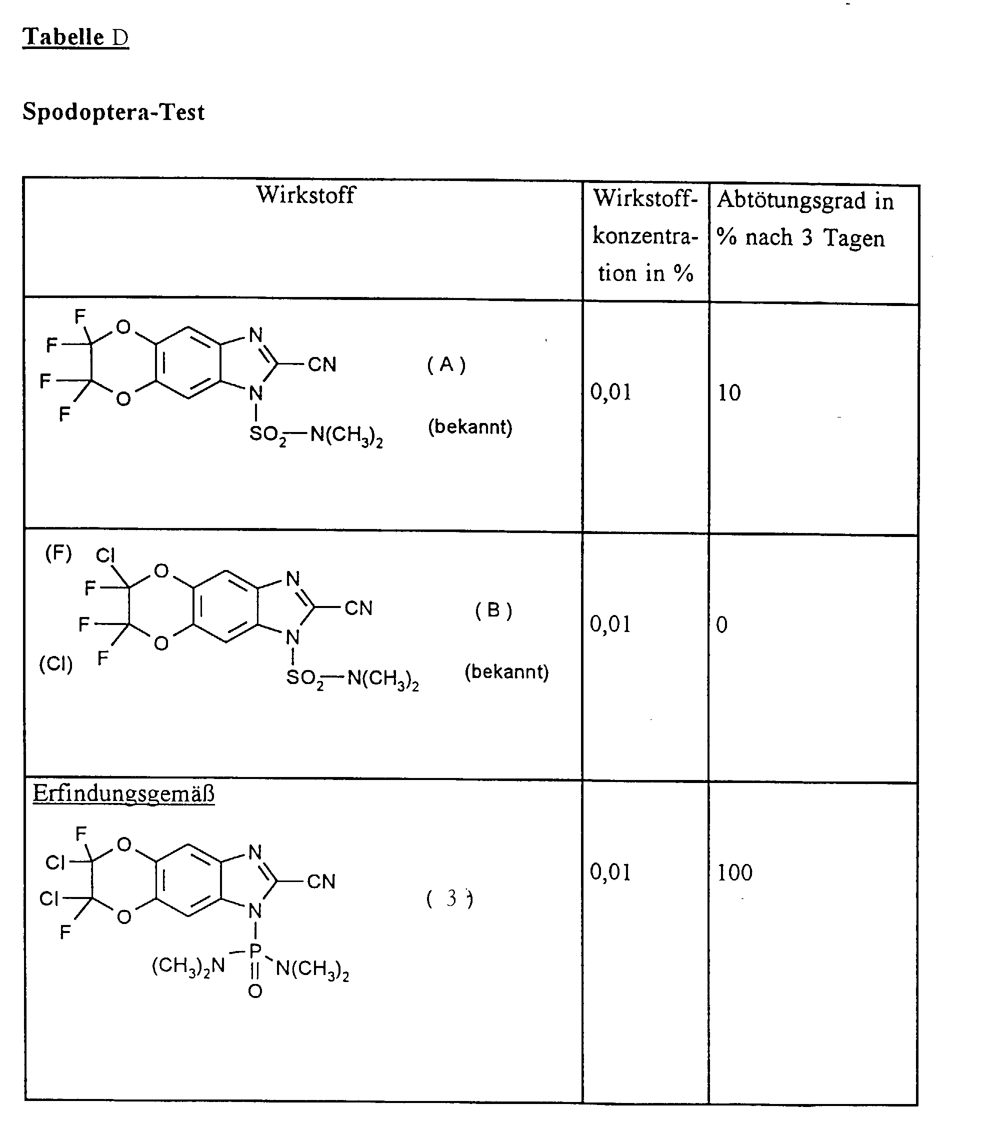

### Beispiel E

### Nephotettix-Test

| | |
|---|---|
| Lösungsmittel | 7 Gewichtsteile Dimethylformamid |
| Emulgator | 1 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Reiskeimlinge (Oryza sativa) werden durch Tauchen in die Wirkstoffzubereitung der gewünschten Konzentration behandelt und mit der Grünen Reiszikade Nephotettixcincticeps besetzt, solange die Blätter noch feucht sind.

Nach der gewünschten Zeit wird der Abtötungsgrad in % bestimmt. Dabei bedeutet 100%, daß alle Reiszikaden abgetötet wurden; 0% bedeutet, daß keine Reiszikaden abgetötet wurden.

In diesem Test zeigen die Verbindungen gemäß den Herstellungsbeispielen 1 und 4 bei einer Wirkstoffkonzentration von 0,01% nach 6 Tagen einen Abtötungsgrad von 100%, während die Vergleichssubstanzen (A) und (B) keine Wirkung aufweisen.

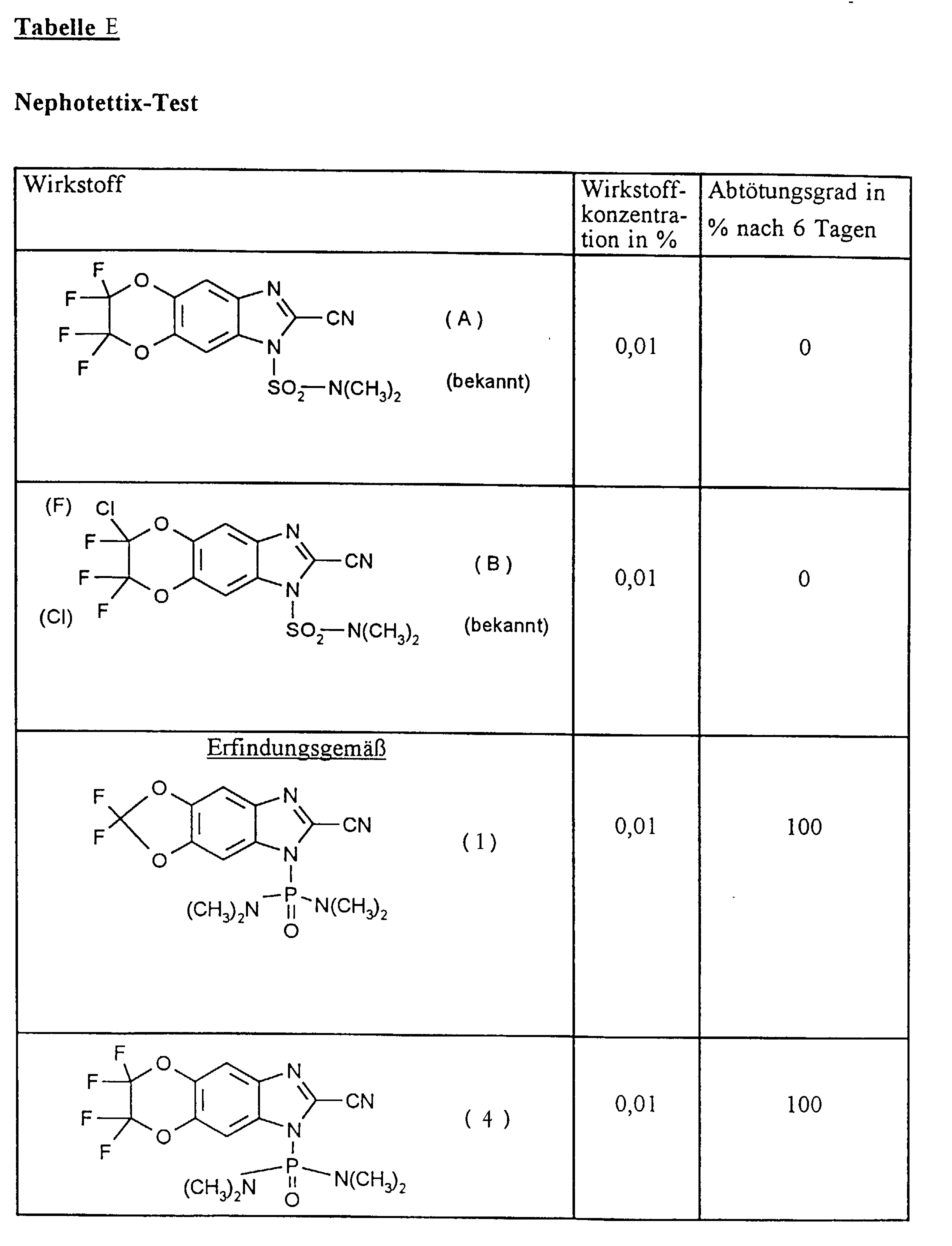

### Beispiel F

### Myzus-Test

| | |
|---|---|
| Lösungsmittel | 7 Gewichtsteile Dimethylformamid |
| Emulgator | 1 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Kohlblätter (Brassica oleracea),die stark von der Pfirsichblattlaus (Myzus persicae) befallen sind, werden durch Tauchen in die Wirkstoffzubereitung der gewünschten Konzentration behandelt

Nach der gewünschten Zeit wird der Abtötungsgrad in % bestimmt. Dabei bedeutet 100%, daß alle Blattläuse abgetötet wurden; 0% bedeutet, daß keine Blattläuse abgetötet wurden.

In diesem Test zeigt die Verbindung gemäß Herstellungsbeispiel 1 bei einer Wirkstoffkonzentration von 0,1% nach 6 Tagen einen Abtötungsgrad von 90%, während die Vergleichssubstanzen (A) und (B) keine Wirkung aufweisen.

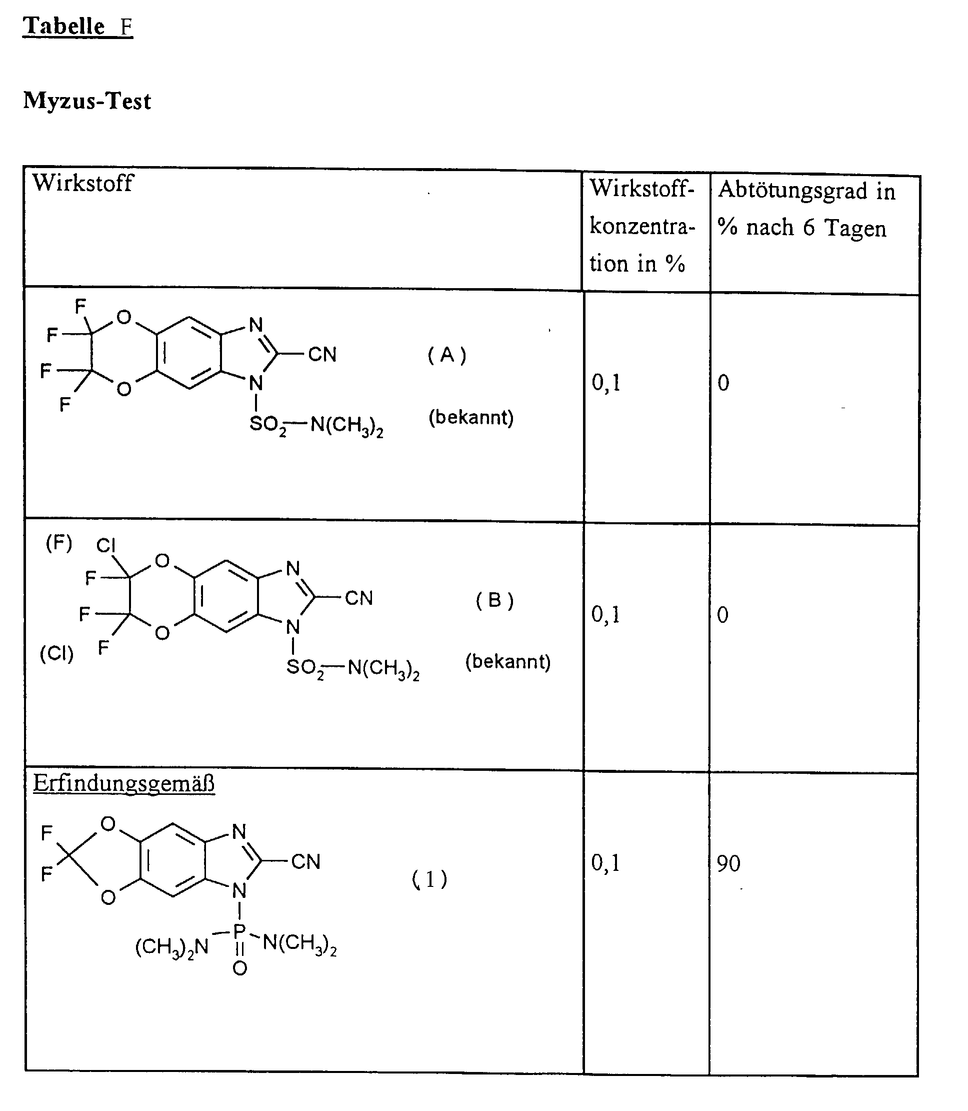

## Patentansprüche

1. Substituierte Benzimidazole der Formel 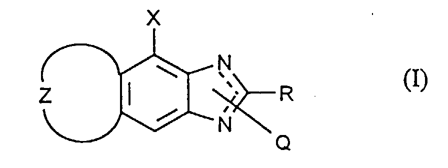 in welcher
R für Cyano oder die Gruppierungen 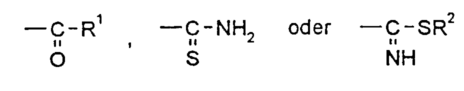 steht, worin
R¹ für geradkettiges oder verzweigtes Alkyl mit 1 bis 4 Kohlenstoffatomen, geradkettiges oder verzweigtes Alkenyl mit 2 bis 4 Kohlenstoffatomen, geradkettiges oder verzweigtes Alkinyl mit 2 bis 4 Kohlenstoffatomen, geradkettiges oder verzweigtes Halogenalkyl mit 1 bis 4 Kohlenstoffatomen und 1 bis 5 Fluor-, Chlor- und/oder Bromatomen oder für geradkettiges oder verzweigtes Halogenalkenyl mit 2 bis 4 Kohlenstoffatomen und 1 bis 5 Fluor-, Chlor- und/oder Bromatomen steht,
R² für geradkettiges oder verzweigtes Alkyl mit 1 bis 4 Kohlenstoffatomen, geradkettiges oder verzweigtes Alkenyl mit 2 bis 4
Kohlenstoffatomen, geradkettiges oder verzweigtes Alkinyl mit 2 bis 4 Kohlenstoffatomen, geradkettiges oder verzweigtes Halogenalkyl mit 1 bis 4 Kohlenstoffatomen und 1 bis 5 Fluor-, Chlor- und/oder
Bromatomen oder für geradkettiges oder verzweigtes Halogenalkenyl mit 2 bis 4 Kohlenstoffatomen und 1 bis 5 Fluor-, Chlor- und/oder Bromatomen steht,
Q für die Gruppierung 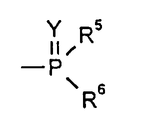 steht, worin
Y für Sauerstoff oder Schwefel steht,
R⁵ und R⁶ unabhängig voneinander für geradkettiges oder verzweigtes Alkyl mit 1 bis 4 Kohlenstoffatomen, geradkettiges oder verzweigtes Alkoxy mit 1 bis 4 Kohlenstoffatomen, geradkettiges oder verzweigtes Alkylthio mit 1 bis 4 Kohlenstoffatomen, geradkettiges oder verzweigtes Alkenyl mit 2 bis 4 Kohlenstoffatomen, geradkettiges oder verzweigtes Alkenyloxy mit 2 bis 4 Kohlenstoffatomen, geradkettiges oder verzweigtes Alkenylthio mit 2 bis 4 Kohlenstoffatomen, geradkettiges oder verzweigtes Alkinyl mit 2 bis 4 Kohlenstoffatomen, geradkettiges oder verzweigtes Alkinyloxy mait 2 bis 4 Kohlenstoffatomen, geradkettiges oder verzweigtes Alkinylthio mit 2 bis 4 Kohlenstoffatomen, Amino, Alkylamino mit 1 bis 4 Kohlenstoffatomen oder für Dialkylamino mit 1 bis 4 Kohlenstoffatomen in jedem Alkylteil stehen,
oder für Phenyl, Phenoxy oder Phenylthio stehen, wobei jeder dieser Reste einfach bis dreifach, gleichartig oder verschieden substituiert sein kann durch Fluor, Chlor, Brom, Alkyl mit 1 bis 4 Kohlenstoffatomen, Halogenalkyl mit 1 bis 4 Kohlenstoffatomen und 1 bis 5 Fluor-, Chlor- und/oder Bromatomen, Alkoxy mit 1 bis 4
Kohlenstoffatomen und/oder Halogenalkoxy mit 1 bis 4 Kohlenstoffatomen und 1 bis 5 Fluor-, Chlor- und/oder Bromatomen, oder für Cycloalkyl mit 3 bis 7 Kohlenstoffatomen, Cycloalkyloxy mit 3 bis 7 Kohlenstoffatomen, Cycloalkylthio mit 3 bis 7 Kohlenstoffatomen, Cycloalkylamino mit 3 bis 7 Kohlenstoffatomen, Di-cycloalkylamino mit 3 bis 7 Kohlenstoffatomen in jedem Cycloalkylteil, N-Phenyl-N-alkyl-amino mit 1 bis 4 Kohlenstoffatomen im Alkylteil, Pyrrolidinyl, Piperidinyl, Pyrrolinyl, Dihydropyridinyl oder Tetrahydropyridinyl stehen, wobei jeder dieser zuvor genannten Reste einfach bis dreifach, gleichartig oder verschieden substituiert sein kann durch Fluor, Chlor, Brom, Alkyl mit 1 bis 4 Kohlenstoffatomen, Alkoxy mit 1 bis 4 Kohlenstoffatomen. und/oder Halogenalkyl mit 1 bis 4 Kohlenstoffatomen und 1 bis 5 Fluor-, Chlor- und/oder Bromatomen, oder
R⁵ und R⁶ gemeinsam mit dem Phosphoratom, an das sie gebunden sind, für einen 5- oder 6-gliedrigen Heterocyclyl-Rest stehen, der ein oder zwei weitere Heteroatome, wie Sauerstoff, Schwefel und/oder Stickstoff, enthalten kann und einfach bis dreifach, gleichartig oder verschieden substituiert sein kann durch Fluor, Chlor, Brom, Alkyl mit 1 bis 4 Kohlenstoffatomen, Alkoxy mit 1 bis 4 Kohlenstoffatomen und/oder Halogenalkoxy mit 1 bis 4 Kohlenstoffatomen und 1 bis 5 Fluor-, Chlor- und/oder Bromatomen,
X für Wasserstoff, Fluor, Chlor oder Brom steht und
Z für die Gruppierungen 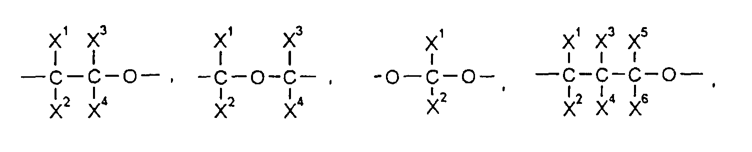 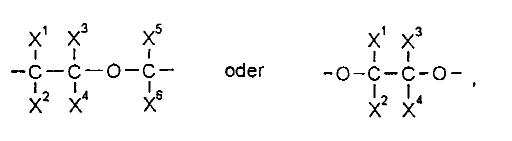 steht, worin
X¹, X³ und X⁵ unabhängig voneinander für Wasserstoff, Fluor, Chlor- oder Brom stehen und
X², X⁴ und X⁶ unabhängig voneinander für Fluor, Chlor oder Brom stehen.

2. Substituierte Benzimidazole der Formel (I) gemäß Anspruch 1, in welcher
R für Cyano oder für die Gruppierungen 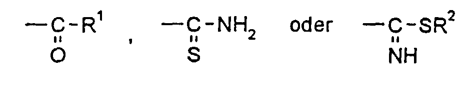 steht, worin
R¹ für Methyl, Ethyl, n- oder i-Propyl, n-,i-, s- oder t-Butyl; Allyl, n- oder s-Butenyl; Propargyl, n- oder s-Butinyl; oder für einfach bis dreifach, gleich oder verschieden durch Fluor und/oder Chlor substituiertes Methyl, Ethyl, Allyl und n- oder s-Butenyl steht,
R² für Methyl, Ethyl, n- oder i-Propyl, n-, i-, s- oder t-Butyl; Allyl, n- oder s-Butenyl; Propargyl, n- oder s-Butinyl; oder für einfach bis dreifach, gleich oder verschieden durch Fluor und/oder Chlor substituiertes Methyl, Ethyl, Allyl und n- oder s-Butenyl steht,
Q für die Gruppierung 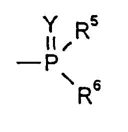 steht, worin
Y für Sauerstoff oder Schwefel steht,
R⁵ und R⁶ unabhängig voneinander für Methyl, Ethyl, n- oder i-Propyl, n-, i-, s- oder t-Butyl; Methoxy, Ethoxy, n- oder i- Propoxy, n-, i-, s- oder t-Butoxy; Methylthio, Ethylthio, n- oder i-Propylthio; Allyl, n- oder s-Butenyl; Allyloxy, n- oder s-Butenyloxy; Allylthio, n- oder s-Butenylthio; Propargyl, n- oder s-Butinyl; Propargyloxy; Propargylthio; Amino; Methylamino, Ethylamino, n- oder i-Propylamino, n-, i-, s- oder t-Butylamino; Dimethylamino, Diethylamino, Di-n- oder i-Propylamino, Methylethylamino, Methyl-n- oder i-Propylamino stehen, oder
für Phenyl, Phenoxy oder Phenylthio, wobei jeder dieser Reste einfach bis dreifach, gleichartig oder verschieden substituiert sein kann durch Fluor, Chlor, Brom, Methyl, Ethyl, n- oder i-Propyl, Methoxy, Ethoxy, n- oder i-Propoxy, Trifluormethyl und/oder Trifluormethoxy stehen, oder für Cyclopropyl, Cyclopentyl, Cyclohexyl, Cyclopropyloxy, Cyclopentyloxy, Cyclohexyloxy, Cyclopropylthio, Cyclopentylthio, Cyclohexylthio, Cyclopropylamino, Cyclopentylamino, Cyclohexylamino, Di-cyclohexylamino, N-Phenyl-N-alkylamino mit 1 bis 3 Kohlenstoffatomen im Alkylteil, 1-Pyrrolidinyl, 1-Pyrrolinyl, 1-Piperidinyl, 1-Dihydropyridinyl und 1-Tetrahydropyridinyl, wobei jeder dieser Reste einfach bis dreifach, gleichartig oder verschieden substituiert sein kann durch Fluor, Chlor, Brom, Methyl, Ethyl, n- oder i-Propyl, Methoxy und/oder Trifluormethyl stehen, oder
R⁵ und R⁶ gemeinsam mit dem Phosphoratom, an das sie gebunden sind, für einen 5- oder 6-gliedrigen Heterocyclyl-Rest, der ein oder zwei weitere Heteroatome, wie Sauerstoff, Schwefel und/oder Stickstoff, enthalten kann und einfach bis dreifach, gleichartig oder verschieden substituiert sein kann durch Methyl, Ethyl, n- oder i-Propyl, Methoxy, Ethoxy, n- oder i-Propoxy, Chlor und/oder Trifluormethyl stehen und
Z für die Gruppierungen -O-CF₂-O-, -O-CF₂-CHF-O-, -O-CHF-CHF-O-, -O-CF₂-CF₂-O-, -O-CF₂-CFCl-O- oder -O-CFCI-CFCI-O-. steht,

3. Substituierte Benzimidazole gemäß Anspruch 1, gekennzeichnet durch die Formeln 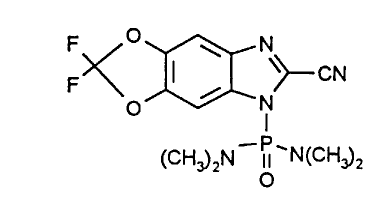 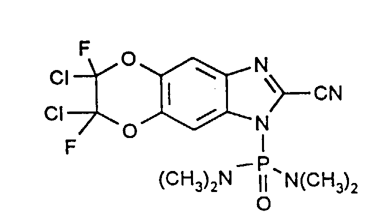 und

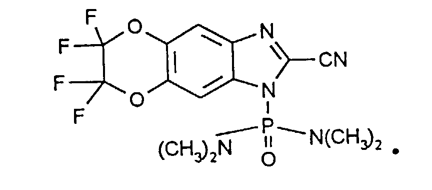

4. Verfahren zur Herstellung von substituierten Benzimidazolen der Formel (I) gemäß Anspruch 1
dadurch gekennzeichnet, daß man Benzimidazol-Derivate der Formel 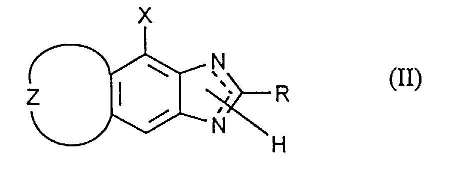 in welcher
R, X und Z die oben angegebenen Bedeutungen haben,
mit Chloriden der Formel
Cl-Q (III)
in welcher
Q die oben angegebene Bedeutung hat,
gegebenenfalls in Gegenwart eines Verdünnungsmittels und gegebenenfalls in Gegenwart einer anorganischen oder organischen Base umsetzt.

5. Schädlingsbekämpfungsmittel, gekennzeichnet durch einen Gehalt an mindestens einem substituierten Benzimidazol der Formel (I) gemäß Anspruch 1.

6. Verwendung von substituierten Benzimidazolen der Formel (I) gemäß Anspruch 1 zur Bekämpfung von Schädlingen.

7. Verfahren zur Herstellung von Schädlingsbekämpfungsmitteln, dadurch gekennzeichnet, daß man substituierte Benzimidazole der Formel (I) gemäß Anspruch 1 mit Streckmitteln und/oder oberflächenaktiven Stoffen vermischt.

## Claims

1. Substituted benzimidazoles of the formula 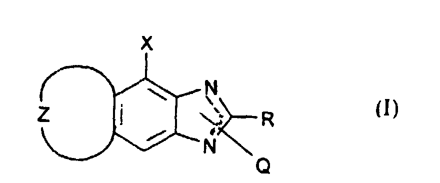 in which
R represents cyano or the groups 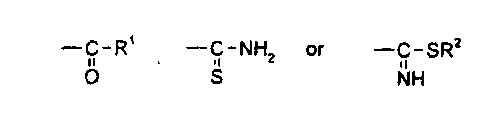 in which
R¹ represents straight-chain or branched alkyl having 1 to 4 carbon atoms, straight-chain or branched alkenyl having 2 to 4 carbon atoms, straight-chain or branched alkinyl having 2 to 4 carbon atoms, straight-chain or branched halogenoalkyl having 1 to 4 carbon atoms and 1 to 5 fluorine, chlorine and/or bromine atoms or represents straight-chain or branched halogenoalkenyl having 2 to 4 carbon atoms and 1 to 5 fluorine, chlorine and/or bromine atoms,
R² represents straight-chain or branched alkyl having 1 to 4 carbon atoms, straight-chain or branched alkenyl having 2 to 4 carbon atoms, straight-chain or branched alkinyl having 2 to 4 carbon atoms, straight-chain or branched halogenoalkyl having 1 to 4 carbon atoms and 1 to 5 fluorine, chlorine and/or bromine atoms or represents straight-chain or branched halogenoalkenyl having 2 to 4 carbon atoms and 1 to 5 fluorine, chlorine and/or bromine atoms,
Q represents the group 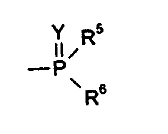 in which
Y represents oxygen or sulphur,
R⁵ and R⁶ independently of one another represent straight-chain or branched alkyl having 1 to 4 carbon atoms, straight-chain or branched alkoxy having 1 to 4 carbon atoms, straight-chain or branched alkylthio having 1 to 4 carbon atoms, straight-chain or branched alkenyl having 2 to 4 carbon atoms, straight-chain or branched alkenyloxy having 2 to 4 carbon atoms, straight-chain or branched alkenylthio having 2 to 4 carbon atoms, straight-chain or branched alkinyl having 2 to 4 carbon atoms, straight-chain or branched alkinyloxy having 2 to 4 carbon atoms, straight-chain or branched alkinylthio having 2 to 4 carbon atoms, amino, alkylamino having 1 to 4 carbon atoms, or represent dialkylamino having 1 to 4 carbon atoms in each alkyl moiety,
or represent phenyl, phenoxy or phenylthio, it being possible for each of these radicals to be substituted from one to three times by identical or different substituents consisting of fluorine, chlorine, bromine, alkyl having 1 to 4 carbon atoms, halogenoalkyl having 1 to 4 carbon atoms and 1 to 5 fluorine, chlorine and/or bromine atoms, alkoxy having 1 to 4 carbon atoms and/or halogenoalkoxy having 1 to 4 carbon atoms and 1 to 5 fluorine, chlorine and/or bromine atoms,
or represent cycloalkyl having 3 to 7 carbon atoms, cycloalkyloxy having 3 to 7 carbon atoms, cycloalkylthio having 3 to 7 carbon atoms, cycloalkylamino having 3 to 7 carbon atoms, di-cycloalkylamino having 3 to 7 carbon atoms in each cycloalkyl moiety, N-phenyl-N-alkyl-amino having 1 to 4 carbon atoms in the alkyl moiety, pyrrolidinyl, piperidinyl, pyrrolinyl, dihydropyridinyl or tetrahydropyridinyl, it being possible for each of these abovementioned radicals to be substituted from one to three times by identical or different substituents consisting of fluorine, chlorine, bromine, alkyl having 1 to 4 carbon atoms, alkoxy having 1 to 4 carbon atoms and/or halogenoalkyl having 1 to 4 carbon atoms and 1 to 5 fluorine, chlorine and/or bromine atoms, or
R⁵ and R⁶, together with the phosphorus atom to which they are attached, represent a 5- or 6-membered heterocyclyl radical which can include one or two further heteroatoms, such as oxygen, sulphur and/or nitrogen, and can be substituted from one to three times by identical or different substituents consisting of fluorine, chlorine, bromine, alkyl having 1 to 4 carbon atoms, alkoxy having 1 to 4 carbon atoms and/or halogenoalkoxy having 1 to 4 carbon atoms and 1 to 5 fluorine, chlorine and/or bromine atoms,
X represents hydrogen, fluorine, chlorine or bromine and
Z represents the groups 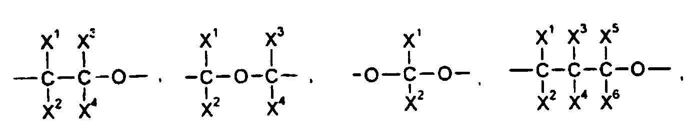 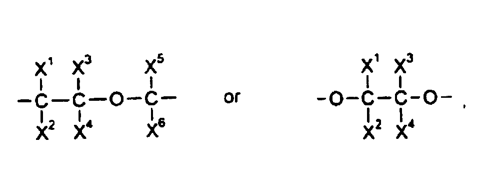 in which
X¹, X³ and X⁵ independently of one another represent hydrogen, fluorine, chlorine or bromine and
X², X⁴ and X⁶ independently of one another represent fluorine, chlorine or bromine.

2. Substituted benzimidazoles of the formula (I) according to Claim 1, in which
R represents cyano or represents the groups 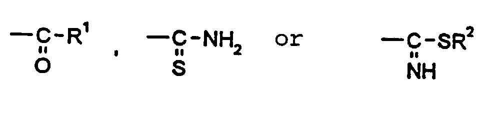 in which
R¹ represents methyl, ethyl, n- or i-propyl, n-, i-, s- or t-butyl; allyl, n- or s-butenyl; propargyl, n- or s-butinyl; or represents methyl, ethyl, allyl and n- or s-butenyl each of which is substituted from one to three times by identical or different substituents selected from fluorine and chlorine,
R² represents methyl, ethyl, n- or i-propyl, n-, i-, s- or t-butyl; allyl, n- or s-butenyl; propargyl, n- or s-butinyl; or represents methyl, ethyl, allyl and n- or s-butenyl each of which is substituted from one to three times by identical or different substituents selected from fluorine and chlorine,
Q represents the group 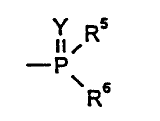 in which
Y represents oxygen or sulphur,
R⁵ and R⁶ independently of one another represent methyl, ethyl, n- or i-propyl, n-, i-, s- or t-butyl; methoxy, ethoxy, n- or i-propoxy, n-, i-, s- or t-butoxy; methylthio, ethylthio, n- or i-propylthio; allyl, n- or s-butenyl; allyloxy, n- or s-butenyloxy; allylthio, n- or s-butenylthio; propargyl, nor s-butinyl; propargyloxy; propargylthio; amino; methylamino, ethylamino, n- or i-propylamino, n-, i-, s- or t-butylamino; dimethylamino, diethylamino, di-n- or -i-propylamino, methylethylamino, methyl-nor -i-propylamino, or
represent phenyl, phenoxy or phenylthio, it being possible for each of these radicals to be substituted from one to three times by identical or different substituents selected from fluorine, chlorine, bromine, methyl, ethyl, n- or i-propyl, methoxy, ethoxy, nor i-propoxy, trifluoromethyl and trifluoromethoxy, or
represent cyclopropyl, cyclopentyl, cyclohexyl, cyclopropyloxy, cyclopentyloxy, cyclohexyloxy, cyclopropylthio, cyclopentylthio, cyclohexylthio, cyclopropylamino, cyclopentylamino, cyclohexylamino, di-cyclohexylamino, N-phenyl-N-alkylamino having 1 to 3 carbon atoms in the alkyl moiety, 1-pyrrolidinyl, 1-pyrrolinyl, 1-piperidinyl, 1-dihydropyridinyl and l-tetrahydropyridinyl, it being possible for each of these radicals to be substituted from one to three times by identical or different substituents selected from fluorine, chlorine, bromine, methyl, ethyl, n- or i-propyl, methoxy and trifluoromethyl, or
R⁵ and R⁶, together with the phosphorus atom to which they are attached, represent a 5- or 6-membered heterocyclyl radical which can include one or two further hetero atoms, such as oxygen, sulphur and/or nitrogen, and can be substituted from one to three times by identical or different substituents selected from methyl, ethyl, n- or i-propyl, methoxy, ethoxy, n- or i-propoxy, chlorine and trifluoromethyl, and
Z represents the groups -O-CF₂-O-, -O-CF₂-CHF-O-, -O-CHF-CHF-O-, -O-CF₂-CF₂-O-, -O-CF₂-CFCl-O- or -O-CFCl-CFCl-O-.

3. Substituted benzimidazoles according to Claim 1, characterized bv the formulae 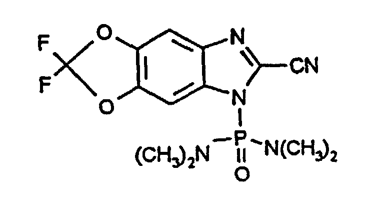 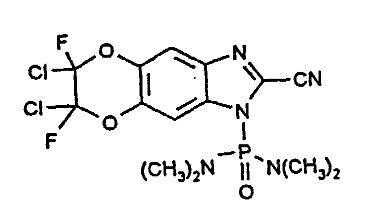 and

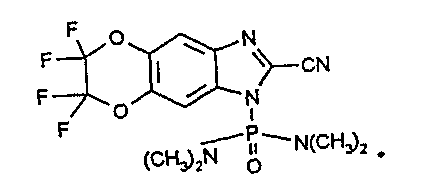

4. Process for the preparation of substituted benzimidazoles of the formula (I) according to Claim 1
characterized in that benzimidazole derivatives of the formula 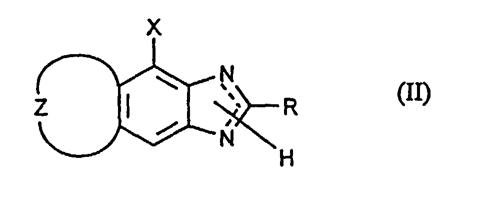 in which
R, X and Z have the meanings given above
are reacted with chlorides of the formula
Cl-Q (III)
in which
Q has the meaning given above,
optionally in the presence of a diluent and optionally in the presence of an inorganic or organic base.

5. Pesticide composition, characterized by a content of at least one substituted benzimidazole of formula (I) according to Claim 1.

6. Use of substituted benzimidazoles of the formula (I) according to Claim 1 for combating pests.

7. Process for producing pesticide compositions, characterized in that substituted benzimidazoles of the formula (I) according to Claim 1 are mixed with extenders and/or surface-active substances.

## Revendications

1. Benzimidazoles substitués répondant à la formule 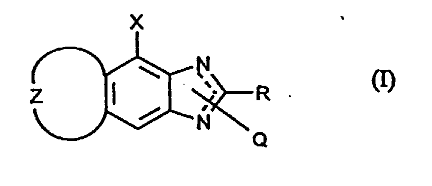 dans laquelle
R représente un groupe cyano ou les groupements 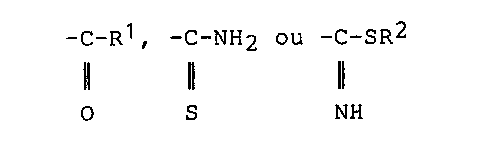 dans lesquels
R¹ représente un groupe alkyle à chaîne droite ou ramifiée contenant de 1 à 4 atomes de carbone, un groupe alcényle à chaîne droite ou ramifiée contenant de 2 à 4 atomes de carbone, un groupe alcynyle à chaîne droite ou ramifiée contenant de 2 à 4 atomes de carbone, un groupe halogénalkyle à chaîne droite ou ramifiée contenant de 1 à 4 atomes de carbone et de 1 à 5 atomes de fluor, de chlore et/ou de brome, ou encore un groupe halogénalcényle à chaîne droite ou ramifiée contenant de 2 à 4 atomes de carbone et de 1 à 5 atomes de fluor, de chlore et/ou de brome,
R² représente un groupe alkyle à chaîne droite ou ramifiée contenant de 1 à 4 atomes de carbone, un groupe alcényle à chaîne droite ou ramifiée contenant de 2 à 4 atomes de carbone, un groupe alcynyle à chaîne droite ou ramifiée contenant de 2 à 4 atomes de carbone, un groupe halogénalkyle à chaîne droite ou ramifiée contenant de 1 à 4 atomes de carbone et de 1 à 5 atomes de fluor, de chlore et/ou de brome, ou encore un groupe halogénalcényle à chaîne droite ou ramifiée contenant de 2 à 4 atomes de carbone et de 1 à 5 atomes de fluor, de chlore et/ou de brome,
Q représente le groupement 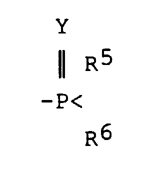 dans lequel
Y représente un atome d'oxygène ou un atome de soufre,
R⁵ et R⁶ représentent, indépendamment l'un de l'autre, un groupe alkyle à chaîne droite ou ramifiée contenant de 1 à 4 atomes de carbone, un groupe alcoxy à chaîne droite ou ramifiée contenant de 1 à 4 atomes de carbone, un groupe alkylthio à chaîne droite ou ramifiée contenant de 1 à 4 atomes de carbone, un groupe alcényle à chaîne droite ou ramifiée contenant de 2 à 4 atomes de carbone, un groupe alcényloxy à chaîne droite ou ramifiée contenant de 2 à 4 atomes de carbone, un groupe alcynyle à chaîne droite ou ramifiée contenant de 2 à 4 atomes de carbone, un groupe alcynyloxy à chaîne droite ou ramifiée contenant de 2 à 4 atomes de carbone, un groupe alcynylthio à chaîne droite ou ramifiée contenant de 2 à 4 atomes de carbone, un groupe amino, un groupe alkylamino contenant de 1 à 4 atomes de carbone ou encore un groupe dialkylamino contenant de 1 à 4 atomes de carbone dans chaque fraction alkyle,
ou encore représentent un groupe phényle, un groupe phénoxy ou un groupe phénylthio, chacun de ces radicaux pouvant porter de un à trois substituants identiques ou différents fluoro, chloro, bromo, alkyle contenant de 1 à 4 atomes de carbone, halogénalkyle contenant de 1 à 4 atomes de carbone et de 1 à 5 atomes de fluor, de chlore et/ou de brome, alcoxy contenant de 1 à 4 atomes de carbone et/ou halogénalcoxy contenant de 1 à 4 atomes de carbone et de 1 à 5 atomes de fluor, de chlore et/ou de brome,
ou représentent un groupe cycloalkyle contenant de 3 à 7 atomes de carbone, un groupe cycloalcoxy contenant de 3 à 7 atomes de carbone, un groupe cycloalkylthio contenant de 3 à 7 atomes de carbone, un groupe cycloalkylamino contenant de 3 à 7 atomes de carbone, un groupe dicycloalkylamino contenant de 3 à 7 atomes de carbone dans chaque fraction cycloalkyle, un groupe N-phényl-N-alkylamino contenant de 1 à 4 atomes de carbone dans la fraction alkyle, un groupe pyrrolidinyle, un groupe pipéridinyle, un groupe pyrrolinyle, un groupe dihydropyridinyle ou un groupe tétrahydropyridinyle, chacun de ces radicaux mentionnés ci-dessus pouvant porter de un à trois substituants identiques ou différents fluoro, chloro, bromo, alkyle contenant de 1 à 4 atomes de carbone, alcoxy contenant de 1 à 4 atomes de carbone et/ou halogénalkyle contenant de 1 à 4 atomes de carbone et de 1 à 5 atomes de fluor, de chlore et/ou de brome, ou bien
R⁵ et R⁶ représentent, ensemble avec l'atome de phosphore auquel ils sont liés, un radical hétérocyclique penta- ou hexagonal qui peut contenir un ou deux hétéroatomes supplémentaires tels qu'un atome d'oxygène, un atome de soufre et/ou un atome d'azote, et qui peut porter de un à trois substituants identiques ou différents fluoro, chloro, bromo, alkyle contenant de 1 à 4 atomes de carbone, alcoxy contenant de 1 à 4 atomes de carbone et/ou halogénalcoxy contenant de 1 à 4 atomes de carbone et de 1 à 5 atomes de fluor, de chlore et/ou de brome,
X représente un atome d'hydrogène, un atome de fluor, un atome de chlore ou un atome de brome, et
Z représente les groupements 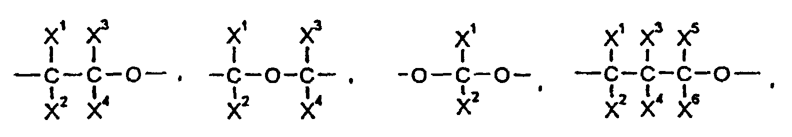 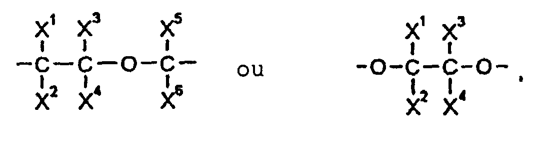 dans lesquels
X¹, X³ et X⁵ représentent, indépendamment l'un de l'autre, un atome d'hydrogène, un atome de fluor, un atome de chlore ou un atome de brome,
X², X⁴ et X⁶ représentent, indépendamment l'un de l'autre, un atome de fluor, un atome de chlore ou un atome de brome.

2. Benzimidazoles substitués répondant à la formule (I) selon la revendication 1, dans laquelle
R représente un groupe cyano ou les groupements 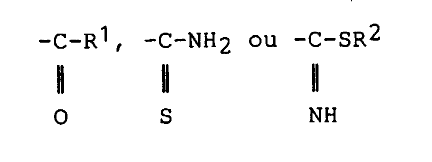 dans lesquels
R¹ représente un groupe méthyle, un groupe éthyle, un groupe n- ou i-propyle, un groupe n-, i-, s- ou t-butyle; un groupe allyle, un groupe n- ou s-butényle; un groupe propargyle, un groupe n- ou s-butynyle; ou encore un groupe méthyle, un groupe éthyle, un groupe allyle et un groupe n- ou s-butényle portant de un à trois substituants fluoro et/ou chloro identiques ou différents,
R² représente un groupe méthyle, un groupe éthyle, un groupe n- ou i-propyle, un groupe n-, i-, s- ou t-butyle; un groupe allyle, un groupe n- ou s-butényle; un groupe propargyle, un groupe n- ou s-butynyle; ou encore un groupe méthyle, un groupe éthyle, un groupe allyle et un groupe n- ou s-butényle portant de un à trois substituants fluoro et/ou chloro identiques ou différents,
Q représente le groupement 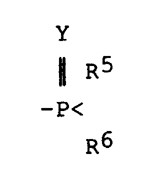 dans lequel
Y représente un atome d'oxygène ou un atome de soufre,
R⁵ et R⁶ représentent, indépendamment l'un de l'autre, un groupe méthyle, un groupe éthyle, un groupe n- ou i-propyle, un groupe n-, i-, s- ou t-butyle; un groupe méthoxy, un groupe éthoxy, un groupe n- ou i-propoxy, un groupe n-, i-, s- ou t-butoxy; un groupe méthylthio, un groupe éthylthio, un groupe n- ou i-propylthio; un groupe allyle, un groupe n- ou s-butényle; un groupe allyloxy, un groupe n- ou s-butényloxy; un groupe allylthio, un groupe n- ou s-buténylthio; un groupe propargyle, un groupe n- ou s-butynyle; un groupe propargyloxy; un groupe propargylthio; un groupe amino; un groupe méthylamino, un groupe éthylamino, un groupe n- ou i-propylamino, un groupe n-, i-, s- ou t-butylamino; un groupe diméthylamino, un groupe diéthylamino, un groupe di-n- ou i-propylamino, un groupe méthyléthylamino, un groupe méthyl-n -ou i-propylamino,
ou encore représentent un groupe phényle, un groupe phénoxy ou un groupe phénylthio, chacun de ces radicaux pouvant porter de un à trois substituants identiques ou différents fluoro, chloro, bromo, méthyle, éthyle, n- ou i-propyle, méthoxy, éthoxy, un groupe n- ou i-propoxy, trifluorométhyle et/ou trifluorométhoxy, ou bien représentent un groupe cyclopropyle, un groupe cyclopentyle, un groupe cyclohexyle, un groupe cyclopropyloxy, un groupe cyclopentyloxy, un groupe cyclohexyloxy, un groupe cyclopropylthio, un groupe cyclopentylthio, un groupe cyclohexylthio, un groupe cyclopropylamino, un groupe cyclopentylamino, un groupe cyclohexylamino, un groupe dicyclohexylamino, un groupe N-phényl-N-alkylamino contenant de 1 à 3 atomes de carbone dans la fraction alkyle, un groupe 1-pyrrolidinyle, un groupe 1-pyrrolinyle, un groupe 1-pipéridinyle, un groupe 1-dihydropyridinyle et un groupe 1-tétrahydropyridinyle, chacun de ces radicaux pouvant porter de un à trois substituants identiques ou différents fluoro, chloro, bromo, méthyle, éthyle, n- ou i-propyle, méthoxy et/ou trifluorométhyle, ou bien
R⁵ et R⁶ représentent, ensemble avec l'atome de phosphore auquel ils sont liés, un radical hétérocyclique penta- ou hexagonal qui peut contenir un ou deux hétéroatomes supplémentaires tels qu'un atome d'oxygène, un atome de soufre et/ou un atome d'azote, et qui peut porter de un à trois substituants identiques ou différents méthyle, éthyle, n- ou i-propyle, méthoxy, éthoxy, n- ou i-propoxy, chloro et/ou trifluorométhyle, et
Z représente les groupements -O-CF₂-O-, -O-CF₂-CHF-O-, -O-CHF-CHF-O-, -O-CF₂-CF₂-O-, -O-CF₂-CFCl-O- ou -O-CFCl-CFCl-O-.

3. Benzimidazoles substitués selon la revendication 1, caractérisés par les formules

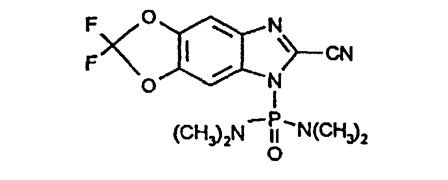

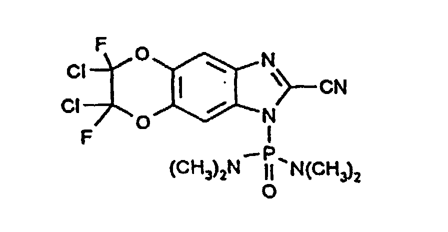

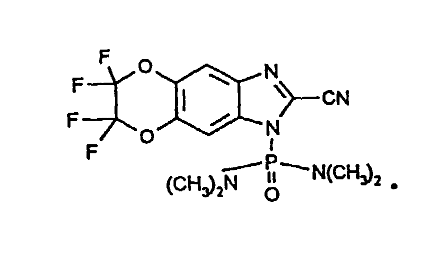

4. Procédé pour la préparation de benzimidazoles substitués répondant à la formule (I) selon la revendication 1,
caractérisé en ce qu'on fait réagir des dérivés de benzimidazole répondant à la formule 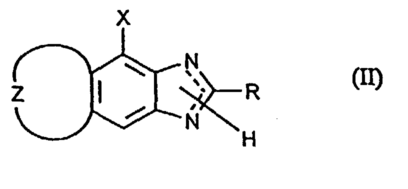 dans laquelle
R, X et Z ont les significations indiquées ci-dessus,
avec des chlorures répondant à la formule
Cl-Q (III)
dans laquelle
Q a la signification indiquée ci-dessus,
le cas échéant en présence d'un diluant et le cas échéant en présence d'une base inorganique ou organique.

5. Agent de lutte contre les parasites, caractérisé par une teneur en au moins un benzimidazole substitué de formule (I) selon la revendication 1.

6. Utilisation de benzimidazoles substitués répondant à la formule (I) selon la revendication 1 pour lutter contre des parasites.

7. Procédé pour la préparation d'agents de lutte contre les parasites, caractérisé en ce qu'on mélange des benzimidazoles substitués répondant à la formule (I) selon la revendication 1 avec des diluants et/ou avec des substances tensioactives.
